# EUROPEAN PATENT APPLICATION

(11) **EP 1 514 946 A1**
(43) Date of publication of application: **16.03.2005**
(21) Application number: 03752914.6
(22) Date of filing: 16.05.2003
(51) Int. Cl.: C12Q 1/68, C12N 15/09, G01N 33/50, G01N 33/15

(54) **METHOD OF DETECTING GENE POLYMORPHISM**

(30) Priority: 17.05.2002 JP 2002143185; 17.10.2002 JP 2002303528
(71) Applicant: Riken, Wako-shi, Saitama 351-0198 (JP); Nakamura, Yusuke, Yokohama-shi, Kanagawa 225-0011 (JP); Sekine, Akihiro, Kunitachi-shi, Tokyo 186-0001 (JP); Iida, Aritoshi, Kawasaki-shi, Kanagawa 211-0014 (JP); Saito, Susumu, Ome-shi, Tokyo 198-0042 (JP)
(72) Inventor: NAKAMURA, Yusuke, Yokohama-shi, Kanagawa 225-0011 (JP); SEKINE, Akihiro, Kunitachi-shi, Tokyo 186-0001 (JP); IIDA, Aritoshi, Kawasaki-shi, Kanagawa 211-0014 (JP); SAITO, Susumu, Ome-shi, Tokyo 198-0042 (JP)
(74) Representative: Glawe, Delfs, Moll & Partner
(86) International application number: PCT/JP2003/006141
(87) International publication number: WO 2003/097877

(57) **Abstract**

A method for detecting a genetic polymorphism(s), comprising creating oligonucleotide probes and/or oligonucleotide primers so that the probes and/or primers contain a polymorphic site(s) present in a gene encoding a receptor or a sequence complementary thereto or so that the polymorphic site(s) is/are contained in the amplified fragment when at least one of said gene encoding the receptor and said sequence complementary thereto is amplified; and detecting at least one genetic polymorphism in a gene of a subject encoding the receptor using the resultant oligonucleotide probes and/or oligonucleotide primers.

## Description

### TECHNICAL FIELD

The present invention relates to information on genetic polymorphisms; a method for detecting information on genetic polymorphisms; a method for evaluating drugs using genetic polymorphisms; and a method for screening for drugs.

### BACKGROUND ART

As physical appearances of human individuals vary infinitely, the human genetic code consisting of three billion (3,000,000,000) base pairs vary at a considerably large number of sites when compared among individuals. These differences in the genetic code are called genetic polymorphisms, and single nucleotide polymorphism is known as a representative polymorphism.

Single nucleotide polymorphism (SNP) means a difference in one DNA letter among individuals. As faces and shapes of human individuals vary infinitely, nucleotide sequences (i.e. genetic code) of individuals vary at a considerably large number of sites. SNPs are classified into cSNP (coding SNP) and gSNP (genome SNP) depending on their locations; cSNP is further classified into sSNP (silent SNP), rSNP (regulatory SNP) and iSNP (intron SNP).

These SNPs are useful as polymorphic markers in searching for those genes which are associated in the development or worsening of diseases; finally, these SNPs directly relates to risk diagnosis of diseases or selection and use of therapeutic drugs in the clinical field. Also, drug development on the basis of evidence obtained using causative substances as target molecules has become the trend of the world. When a drug is administered to patients with the same disease, their responsiveness is diverse. Some patients show remarkable effect; some patients show low effect; and some patients show no effect. Thus, responsiveness to a drug varies greatly depending on the patient. Even if the conditions of patients are the same and diagnosed as the same disease, the routes which have caused that disease may be different; or the ability of the drug to bind to its receptor, the signal transduction ability of the receptor, or the expression level of the receptor itself may vary greatly among patients. Therefore, it is desired to select an appropriate drug and develop an appropriate therapeutic method against a target disease based on genetic polymorphisms such as SNPs (i.e. the so-called personalized medicine is desired).

In addition to responsiveness to drugs, the problem of strong side effect which sometimes might be lethal is also one of the major problems that medical staffs should address. Even if there is no excessive administration caused by prescription error or the like, unexpected, lethal side effect might occur. Therefore, with respect to responsiveness to a drug, it is not sufficient to consider the metabolism and delivery of the drug; it is desired that individual difference in the responsiveness of the drug's receptor and the sensitivities of those receptors associated with side effect should be determined taking into account genetic polymorphisms such as SNPs.

### DISCLOSURE OF THE INVENTION

It is an object of the present invention to provide a method for detecting information on genetic polymorphism; a method for evaluating the efficacy and safety of drugs based on the information; and a method for screening for drugs.

As a result of extensive and intensive researches toward the solution of the above problem, the present inventors have succeeded in establishing a method which comprises detecting genetic polymorphisms in a gene encoding a receptor and evaluating with the resultant information the sensitivity of a drug and the occurrence of side effect through the receptor that was believed to be a non-target of the drug. Thus, the present invention has been achieved.

The present invention is as described below.
(1) A method for detecting a genetic polymorphism(s), comprising creating oligonucleotide probes and/or oligonucleotide primers so that the probes and/or primers contain a polymorphic site(s) present in a gene encoding a receptor or a sequence complementary thereto or so that the polymorphic site(s) is/are contained in the amplified fragment when at least one of the gene encoding the receptor and the sequence complementary thereto is amplified; and detecting at least one genetic polymorphism in a gene of a subject encoding the receptor using the resultant oligonucleotide probes and/or oligonucleotide primers.
(2) A method for detecting a genetic polymorphism(s) comprising creating oligonucleotide probes and/or oligonucleotide primers so that the probes and/or primers contain a polymorphic site(s) present in a gene encoding a receptor or a sequence complementary thereto or so that the polymorphic site(s) is/are contained in the amplified fragment when at least one of the gene encoding the receptor and the sequence complementary thereto is amplified; and detecting at least one genetic polymorphism in a gene of a subject encoding the receptor using the resultant oligonucleotide probes and/or oligonucleotide primers; wherein the polymorphic site is at least one of the polymorphic sites present in the nucleotide sequences as shown in SEQ ID NOS: 1 through 1168 or sequences complementary thereto.
(3) A method for detecting a genetic polymorphism(s) comprising creating oligonucleotide probes and/or oligonucleotide primers so that the probes and/or primers contain a polymorphic site(s) present in a gene encoding a receptor or a sequence complementary thereto or so that the polymorphic site(s) is/are contained in the amplified fragment when at least one of the gene encoding the receptor and the sequence complementary thereto is amplified; and detecting at least one genetic polymorphism in a gene of a subject encoding the receptor using the resultant oligonucleotide probes and/or oligonucleotide primers; wherein the oligonucleotide probe and/or oligonucleotide primer is at least one selected from a group consisting of probes and primers having a polymorphic site-containing at least 13 nucleotide sequence within the nucleotide sequences as shown in SEQ ID NOS: 1 through 1168 or a sequence complementary to the polymorphic site-containing at least 13 nucleotide sequence.
   The length of the above-described oligonucleotide probe and/or oligonucleotide primer may be from 13 to 60 nucleotides.
(4) In the methods described in (1) to (3) above, the information as shown in Table 1 (e.g. the sequence information of SEQ ID NOS: 1-1168 as shown in Table 1) may be used as information of polymorphic sites. As a specific example of the above-described oligonucleotide probe and/or oligonucleotide primer containing a polymorphic site, a probe and/or primer may be given which is created so that the nucleotide positioned at its 5' or 3' end or its central part is the polymorphic site. Also included in the present invention as an oligonucleotide probe containing a polymorphic site is an oligonucleotide probe which is composed of two fragments being linked to each other, wherein one fragment is hybridizable to a gene encoding a receptor or a sequence complementary thereto; the other fragment is not hybridizable thereto; and the polymorphic site is positioned at the 5' or 3' end of the hybridizable fragment.
   In the present invention, the types of genetic polymorphisms are not particularly limited. For example, single-nucleotide polymorphism, polymorphism caused by deletion, substitution or insertion of a plurality of nucleotides, or VNTR or microsatellite polymorphism may be enumerated.
(5) A method for evaluating a drug, comprising evaluating the efficacy and safety of the drug intermediated by the receptor from the detection results obtained by any one of the methods of (1) to (4) above.
(6) A method for evaluating a drug, comprising evaluating the degree of sensitivity of the drug intermediated by the receptor from the detection results obtained by any one of the methods of (1) to (4) above.
(7) A method for selecting drugs, comprising selecting a drug to be used using the evaluation obtained by the method of (5) or (6) above.
(8) A method for selecting drugs, comprising comparing information about a polymorphism(s) in a gene encoding a receptor or a sequence complementary thereto with information about a polymorphism(s) in a gene encoding the receptor or a sequence complementary thereto obtained from a subject; analyzing individual differences regarding the efficacy and/or safety of drugs intermediated by the receptor; and selecting a drug to be used and/or a dose of the drug from the analysis results obtained.
(9) In the detection methods of (1) to (4) above, the evaluation methods of (5) and (6) above, or the selection methods of (7) and (8), at least one selected from the group consisting of CD20, CD33, CSF3R, IL1R1, IL1R2, IL2R, HER2, IFNAR1, PGR, ACTH, ICAM1, VCAM1, ITGB2, PTGDR, PTGER1, PTGER2, PTGER3, PTGFR, GNA12, TBXA2R, BLTR2, CYSLT1, CYSLT2, PTAFR, BDKRB1, BDKRB2, ADRB1, ADRB2, HRH1, HRH2, HRH3, HTR3A, AGTR1, AGTRL1, AGTR2, AVPR1A, AVPR2, PTGIR, DRD1, ITGA2B, FOLR1, TNFR1, ADORA1, ADORA2A, ADORA2B, ADORA3, AVPR1B, ADRA1A, ADRA2A, ADRA2B, EDG1, EDG4, EDG5, GPR1, GPR2, GPR3, GPR4, GPR10, MC1R, MC2R, MC3R, MC4R, OXTR, SSTR1 and SSTR3 may be used as a receptor.
(10) An oligonucleotide created so that it contains a polymorphic site present in a gene encoding a receptor or a sequence complementary thereto.
(11) An oligonucleotide created so that it contains a polymorphic site present in a gene encoding any receptor selected from the group consisting of CD20, CD33, CSF3R, IL1R1, IL1R2, IL2R, HER2, IFNAR1, PGR, ACTH, ICAM1, VCAM1, ITGB2, PTGDR, PTGER1, PTGER2, PTGER3, PTGFR, GNA12, TBXA2R, BLTR2, CYSLT1, CYSLT2, PTAFR, BDKRB1, BDKRB2, ADRB1, ADRB2, HRH1, HRH2, HRH3, HTR3A, AGTR1, AGTRL1, AGTR2, AVPR1A, AVPR2, PTGIR, DRD1, ITGA2B, FOLR1, TNFR1, ADORA1, ADORA2A, ADORA2B, ADORA3, AVPR1B, ADRA1A, ADRA2A, ADRA2B, EDG1, EDG4, EDG5, GPR1, GPR2, GPR3, GPR4, GPR10, MC1R, MC2R, MC3R, MC4R, OXTR, SSTR1 and SSTR3, or a sequence complementary thereto.
(12) The oligonucleotide of (10) or (11) above is created, for example, so that the nucleotide positioned at its 5' or 3' end or its central part is the polymorphic site. Alternatively, the above-mentioned oligonucleotide containing a polymorphic site may be created so that the oligonucleotide is composed of two fragments being linked to each other, wherein one fragment is hybridizable to the gene encoding a receptor or the sequence complementary thereto; the other fragment is not hybridizable thereto; and the polymorphic site is positioned at the 5' or 3' end of the hybridizable fragment. As more specific examples of the oligonucleotide of the invention, oligonucleotides containing at least one polymorphic site present in the nucleotide sequences as shown in SEQ ID NOS: 1-1168 or sequences complementary thereto may be given. These oligonucleotides may oligonucleotides with a length of 13 to 35 nucleotides, or oligonucleotides consisting of an at least 13 nucleotide sequence containing the 21st nucleotide in any one of the nucleotide sequences as shown in SEQ ID NOS: 1-1168 or a sequence complementary to the at least 13 nucleotide sequence. Oligonucleotides selected from the group consisting of the nucleotide sequences as shown in SEQ ID NOS: 1-1168 or sequences complementary thereto are also included in the present invention.
(13) An oligonucleotide which is designed in a genomic DNA region containing a polymorphic site in any of the nucleotide sequences as shown in SEQ ID NOS: 1 through 1168 or sequences complementary thereto so that it is located within 1000 bp of the polymorphic site toward the 5' and/or 3' end of the genomic DNA region, and which has a length of 13-60 nucleotides.
(14) A microarray wherein the oligonucleotide of (12) or (13) above is immobilized on a support.
(15) A genetic polymorphism detection kit comprising the oligonucleotide of (12) or (13) above and/or the microarray of (14) above.

Hereinbelow, the present invention will be described in more detail.

The present invention relates to a method for detecting genetic polymorphisms in a subject using genetic polymorphism information on a receptor. The present invention is also characterized by analyzing the absence/presence or intensity of the efficacy and safety of the drug intermediated by the receptor; based on the analysis result, the relation between the disease and the drug is evaluated. Even when a plurality of patients suffer from the same disease, it is quite often that genetic polymorphism information varies by individual patient. Therefore, difference in receptor sensitivity against a drug is drawn out from genetic polymorphism information different among individuals; then, the efficacy and/or safety of the drug (i.e. the efficacy of the specific drug is recognized or not recognized when a patient has such and such genetic polymorphism information, or side effect occurs frequently or seldom when a patient has such and such genetic polymorphism information) is evaluated. From these results, it becomes possible to determine what drug should be used for a specific disease, and to administer a drug suitable for an individual patient (tailored medicine) based on his/her genetic polymorphism information.

### 1. Genetic Polymorphism

Genetic polymorphism includes single nucleotide polymorphism, insertion/deletion polymorphism, and polymorphism caused by difference in the number of repetition of a nucleotide sequence. Generally, single nucleotide polymorphism (SNP) means a polymorphism caused by substitution of one specific nucleotide with other nucleotide in a gene or its complementary strand (complementary sequence) region. In the present invention, however, the term SNP also includes the polymorphism caused by substitution above as well as a polymorphism caused by deletion of the nucleotide and a polymorphism caused by addition of one more nucleotide to the nucleotide.

Insertion/deletion type polymorphism means a polymorphism caused by deletion or insertion of a plurality of nucleotides (e.g. two to several ten nucleotides). Sometimes, several hundred to several thousand nucleotides may be deleted or inserted. The polymorphism caused by difference in the number of repetition of a nucleotide sequence has repetition of a sequence of two to several ten nucleotides, and the number of this repetition varies among individuals. Those polymorphisms where the repeat unit consists of several to several ten nucleotides are called VNTR (variable number of tandem repeats) polymorphism, and those polymorphisms where the repeat unit consists of about two to four nucleotides are called microsatellite polymorphism. In VNTR or microsatellite polymorphisms, the number of such repetition is different among individuals' alleles, which results in acquisition of variation.

It should be noted here that information on genetic polymorphisms includes both polymorphism information in genes and polymorphism information in the complementary sequences thereto. The term "complementary strand" or "complementary sequence" refers to a polynucleotide having the relationship of the base pairing rules. For example, a sequence 5'-A-G-T-3' is complementary to a sequence 3'-T-C-A-5'. Complementation may be partial (i.e. only a certain number of nucleotides in a polynucleotide are matching according to the base pairing rules), or the entire sequence may be completely complementary. Such degree of complementation significantly influences upon the efficacy and intensity of hybridization. This is particularly important in amplification reaction and detection methods using the binding between polynucleotides.

The complementation of nucleotide sequences means that when one sequence of oligonucleotide is aligned with other sequence so that the 5' end of the former makes a pair with the 3' end of the latter, the sequences of oligonucleotides are antiparallel to each other. It is not necessary that complementation is complete; double helix is stable even if it contains mismatched base pairs or not matched bases. One of ordinary skill in the art could experientially determine the stability of double helix considering, for example, the lengths of oligonucleotides, nucleotide compositions and sequences of oligonucleotides, ion intensity, and the presence of mismatched base pairs, etc.

### 2. Receptors

"Receptor" is a generic term for receivers which respond to specific ligands such as hormones, autacoids, neurotransmitters, etc. As representative receptors, cell membrane receptors and nuclear receptors are known. Some drugs inhibit or antagonize the binding of a specific ligand to such a receptor, or bind to the receptor in the same manner as its ligand does, thus stimulating the signal transduction for which the receptor is responsible. Therefore, when the ability of the ligand to bind to the receptor, the signal transduction ability of the receptor, or the expression level of the receptor itself is influenced by genetic polymorphisms, individual difference occurs in the efficacy or side effect of the drug.

In the present invention, examples of receptors which are expressed by target genes of genetic polymorphism analysis include the following receptors.
CD20, CD33, CSF3R, IL1R1, IL1R2, IL2R, HER2, IFNAR1, PGR, ACTH, ICAM1, VCAM1, ITGB2, PTGDR, PTGER1, PTGER2, PTGER3, PTGFR, GNA12, TBXA2R, BLTR2, CYSLT1, CYSLT2, PTAFR, BDKRB1, BDKRB2, ADRB1, ADRB2, HRH1, HRH2, HRH3, HTR3A, AGTR1, AGTRL1, AGTR2, AVPR1A, AVPR2, PTGIR, DRD1, ITGA2B, FOLR1, TNFR1, ADORA1, ADORA2A, ADORA2B, ADORA3, AVPR1B, ADRA1A, ADRA2A, ADRA2B, EDG1, EDG4, EDG5, GPR1, GPR2, GPR3, GPR4, GPR10, MC1R, MC2R, MC3R, MC4R, OXTR, SSTR1 and SSTR3.

Receptors are receivers which individually control only the signal transduction by a specific ligand.
(1) CD20 represents the receptor for CD20 antigen.
(2) CD33 represents the receptor for CD33 antigen.
(3) CSF3R represents the receptor for colony stimulating factor 3.
(4) IL1R1 represents a receptor for interleukin-1.
(5) IL1R2 represents a receptor for interleukin-1.
(6) IL2R represents the receptor for interleukin-2.
(7) HER2 represents the receptor for c-erb-B-2.
(8) IFNAR1 represents a receptor for interferon alpha, beta and omega.
(9) PGR represents the receptor for progesterone.
(10) ACTH represents the receptor for melanocortin 2.
(11) ICAM1 represents the receptor for human intercellular adhesion molecule 1 (CD54).
(12) VCAM1 represents the receptor for vascular cell adhesion molecule 1.
(13) ITGB2 represents the receptor for integrin, beta 2 [antigen CD18 (p95), lymphocyte function-associated antigen 1; macrophage antigen 1 (mac-1) beta subunit].
(14) PTGDR represents the receptor for prostaglandin D2.
(15) PTGER1 represents the receptor for prostaglandin E1.
(16) PTGER2 represents the receptor for prostaglandin E2.
(17) PTGER3 represents the receptor for prostaglandin E3.
(18) PTGFR represents the receptor for prostaglandin F.
(19) GNA12 represents the receptor for thromboxane A2.
(20) TBXA2R represents the receptor for thromboxane A2.
(21) BLTR2 represents the receptor for leukotriene B4.
(22) CYSLT1 represents a receptor for cysteinyl leukotriene.
(23) CYSLT2 represents a receptor for cysteinyl leukotriene.
(24) PTAFR represents the receptor for platelet-activating factor.
(25) BDKRB1 represents a receptor for bradykinin.
(26) BDKRB2 represents a receptor for bradykinin.
(27) ADRB1 represents the receptor for catecholamine beta-1.
(28) ADRB2 represents the receptor for catecholamine beta-2.
(29) HRH1 represents histamine H1 receptor.
(30) HRH2 represents histamine H2 receptor.
(31) HRH3 represents histamine H3 receptor.
(32) HTR3A represents the receptor for 5-hydroxytryptamine (serotonin).
(33) AGTR1 represents angiotensin receptor 1.
(34) AGTRL1 represents an angiotensin-like receptor.
(35) AGTR2 represents a receptor for angiotensin.
(36) AVPR1A represents a receptor for arginine vasopressin.
(37) AVPR2 represents a receptor for arginine vasopressin.
(38) PTGIR represents the receptor for prostaglandin I2 (prostacyclin).
(39) DRD1 represents a receptor for dopamine.
(40) ITGA2B represents the receptor for integrin, alpha 2b (platelet glycoprotein IIb of IIb/IIIa complex, antigen CD41B).
(41) FOLR1 represents folate receptor 1.
(42) TNFR1 represents tumor necrosis factor receptor 1 (Accession No.: AC006057.5).
(43) ADORA1 represents adenosine A1 receptor.
(44) ADORA2A represents adenosine A2 receptor.
(45) ADORA2B represents adenosine A2B receptor.
(46) ADORA3 represents adenosine A3 receptor.
(47) AVPR1B represents arginine vasopressin receptor 1B.
(48) ADRA1A represents adrenergic alpha-1A receptor.
(49) ADRA2A represents adrenergic alpha-2A receptor.
(50) ADRA2B represents adrenergic alpha-2B receptor.
(51) EDG1 represents endothelial differentiation, sphingolipid G-protein-coupled receptor, 1.
(52) EDG4 represents endothelial differentiation, sphingolipid G-protein-coupled receptor, 4.
(53) EDG5 represents endothelial differentiation, sphingolipid G-protein-coupled receptor, 5.
(54) GPR1 represents G protein-coupled receptor 1.
(55) GPR2 represents G protein-coupled receptor 2.
(56) GPR3 represents G protein-coupled receptor 3.
(57) GPR4 represents G protein-coupled receptor 4.
(58) GPR10 represents G protein-coupled receptor 10.
(59) MC1R represents melanocortin 1 receptor.
(60) MC2R represents melanocortin 2 receptor.
(61) MC3R represents melanocortin 3 receptor.
(62) MC4R represents melanocortin 4 receptor.
(63) OXTR represents oxytocin receptor.
(64) SSTR1 represents somatostatin receptor 1.
(65) SSTR3 represents somatostatin receptor 3.

### 3. Information on Genetic Polymorphisms

Information on genetic polymorphisms may be obtained by conventional methods for detecting genetic polymorphisms. For example, the sequencing method, the PCR method, fragment length polymorphism assay, hybridization methods using an allele-specific oligonucleotide as a template (e.g. TaqMan PCR method, the invader method, the DNA chip method), methods using primer extension reaction, the sequencing method, MALDI-TOF/MS method, the DNA chip method, and the like may be employed. The PCR method and the sequencing method may be used for detecting any type of genetic polymorphism. The other methods may be used mainly for detecting SNPs.

TaqMan PCR is a method using PCR reaction with a fluorescence-labeled, allele-specific oligo(s) and Taq DNA polymerase (Livak, K.J. Genet. Anal. 14, 143 (1999); Morris T. et al., J. Clin. Microbiol. 34, 2933 (1996)). The invader method is a method in which the hybridization of two reporter probes specific to respective alleles of SNP and one invader probe to the template DNA is combined with DNA cleavage by an enzyme having a special endonuclease activity of cleaving upon recognition of DNA structure (for example, see Livak, K. J. Biomol. Eng. 14, 143-149 (1999); Morris T. et al., J. Clin. Microbiol. 34, 2933 (1996); Lyamichev, V. et al., Science, 260, 778-783 (1993)).

As methods using primer extension reaction, SniPer method may be employed, for example. The basic principle of SniPer method is a technique called RCA (rolling circle amplification) method in which DNA polymerase moves on a circular single-stranded DNA as a template to thereby synthesize a complementary strand thereto continuously. According to this method, SNP may be judged by detecting the presence or absence of a coloring reaction that occurs when DNA amplification takes place (Lizardi, P. M. et al., Nature Genet., 19, 225-232 (1998); Piated, A. S. et al., Nature Biotech., 16, 359-363 (1998)).

The sequencing method refers to methods in which polymorphism-containing areas are amplified by PCR and the DNA sequences of the amplified products are sequenced with Dye Terminator or the like to thereby analyze the frequency of genetic polymorphisms (especially SNPs).

MALDI-TOF/MS method is a method using a mass spectrometer. Basically, this is a method for SNP genotyping utilizing the difference in mass of different nucleotides. There are methods using PCR amplification and methods using multiplex (Haff, L.A., Smirnov, I.P., Genome Res., 7, 378-(1997); Little, D.P. et al. Eur. J. Clinica. Chem. Clin. Biochem., 35, 545- (1997); Ross, P., et al. Nat Biotechnol., 16, 1347- (1998)).

The DNA chip method is a method in which a large variety of DNA probes are aligned and immobilized on a baseboard such as glass; then, hybridization of a labeled DNA is performed thereon; and perfect match and one-nucleotide-mismatch are detected discriminably by using a method of detecting the label signal (such as fluorescence) on the probe.

The information on genetic polymorphisms, in particular information on SNPs, which may be used in the method of the present invention is as shown in Table 1 below.

In Table 1, the "Designation of Gene" column shows the designations of the genes encoding receptors. The nucleotides expressed with capital letters in the "Sequence" column (i.e. the nucleotides at position 21 in the "Sequence" column) are the polymorphic information. The sequences in this Table basically represent 20 nucleotides each before and after the SNP. However, some sequences have an additional polymorphism(s) in the 20 nucleotides before or after the polymorphic site at position 21. For example, the "T/C" at position 16 in No. 9 of CD20 (SEQ ID NO: 9), or the "T/C" at position 5 in No. 10 of IL1R (SEQ ID NO: 57) is also a polymorphism. The two nucleotides on both sides of the mark "/" represent a homozygous or heterozygous SNP of the nucleotide. For example, "A/G" means that the allele is A/A or G/G homozygote or A/G heterozygote. However, the nucleotide in parentheses [e.g. (A) in No. 12 of CSF3R: SEQ ID NO: 46] represents a polymorphism caused by insertion. The mark of open triangle (e.g. see No. 37 of IL1R2: SEQ ID NO: 133) means a polymorphism caused by deletion of one or more nucleotides. The nucleotide in parentheses provided with a number means that the nucleotide in the parentheses is repeated that number of times. For example, "(C) 8-10" appearing in SEQ ID NO: 43 (Table 1, No. 9 of CSF3R) means a sequence where C is repeated from 8 to 10 times. It should be noted that the term "position 21" used in explaining locational relations in the nucleotide sequence described in the "Sequence" column in Table 1 means the location of a genetic polymorphism site. Therefore, in the case of deletion SNP (open triangle), the deleted, imaginary nucleotide is the "position 21". In the case where a plurality of nucleotides are present at the polymorphic site, a group of those nucleotides is the "position 21". For example, in the case of No. 18 of VCAM1 (SEQ ID NO: 249), the polymorphic site "GCAG" or the deleted site is the position 21; in the case of No. 41 of IL1R (SEQ ID NO: 89), the polymorphic site "(A) 9-12" is the position 21.

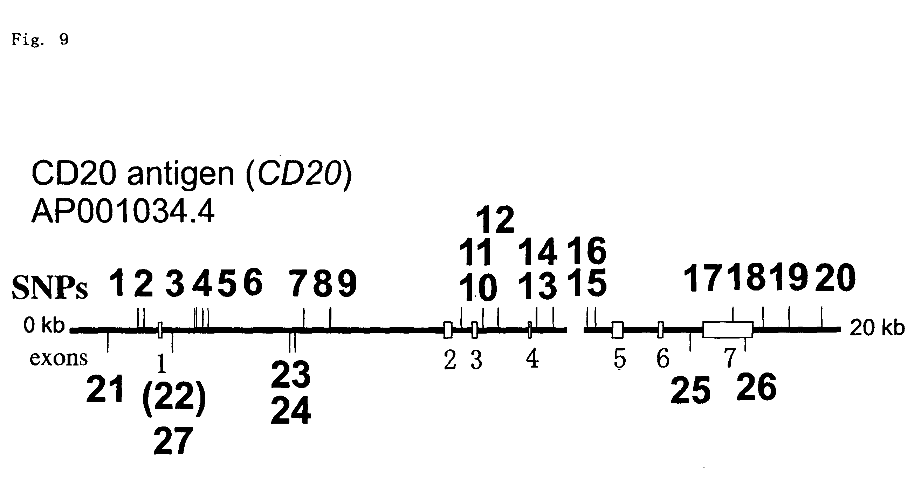

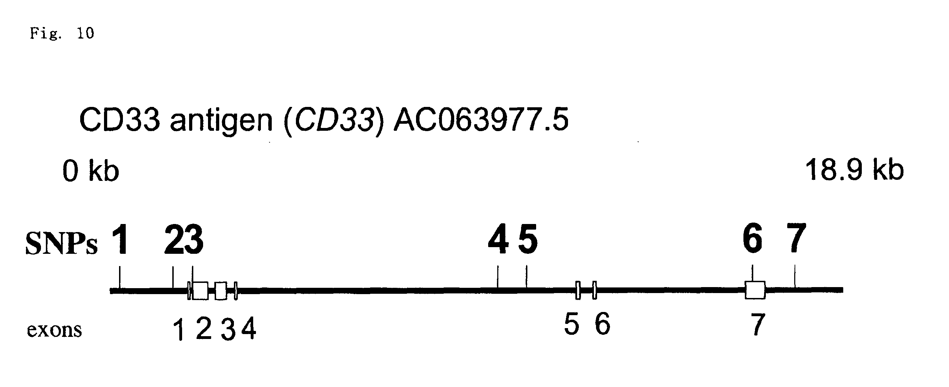

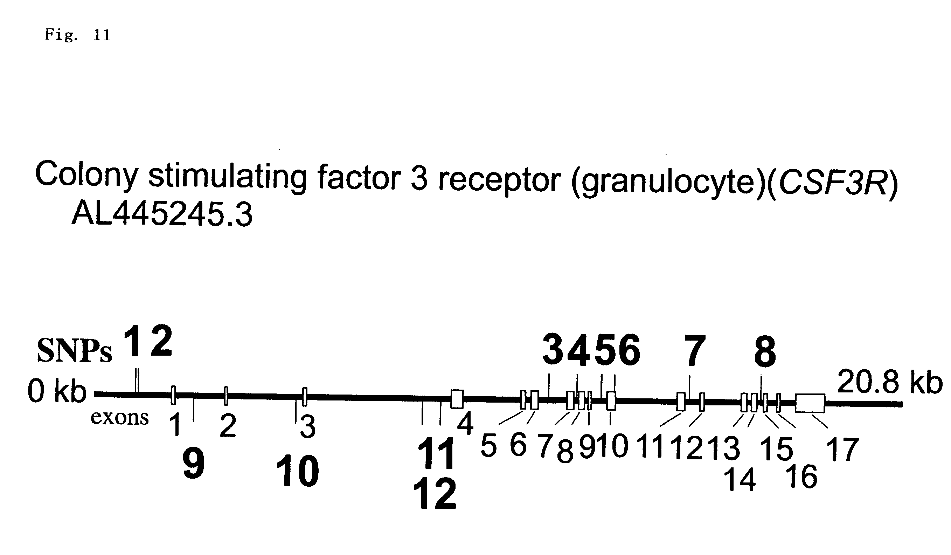

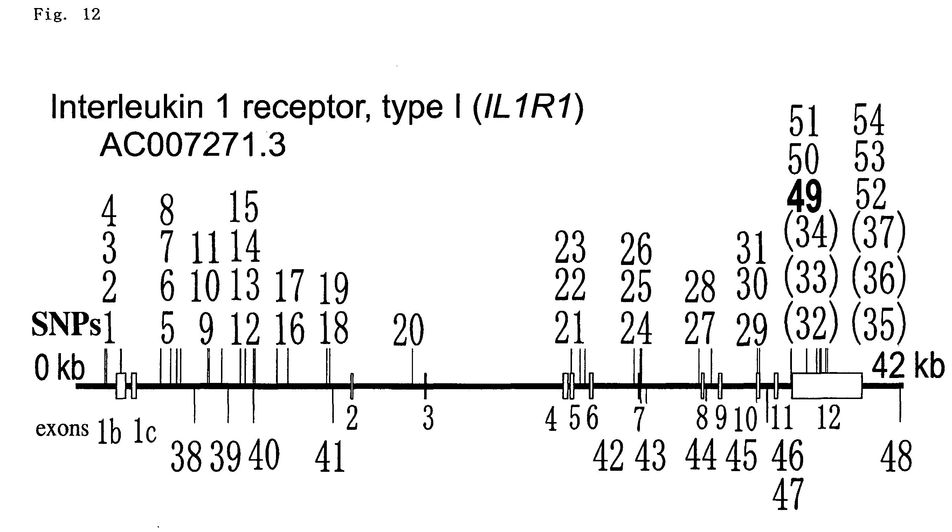

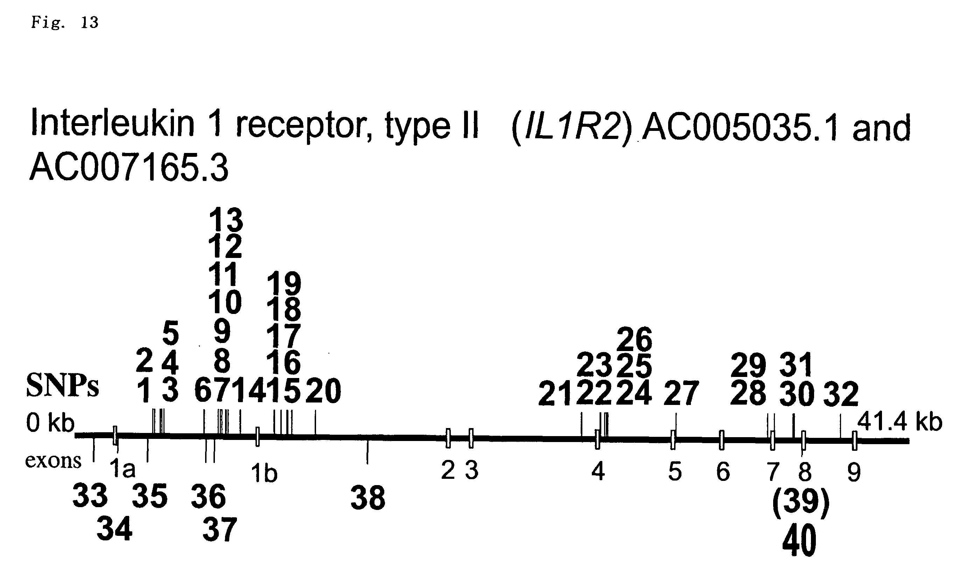

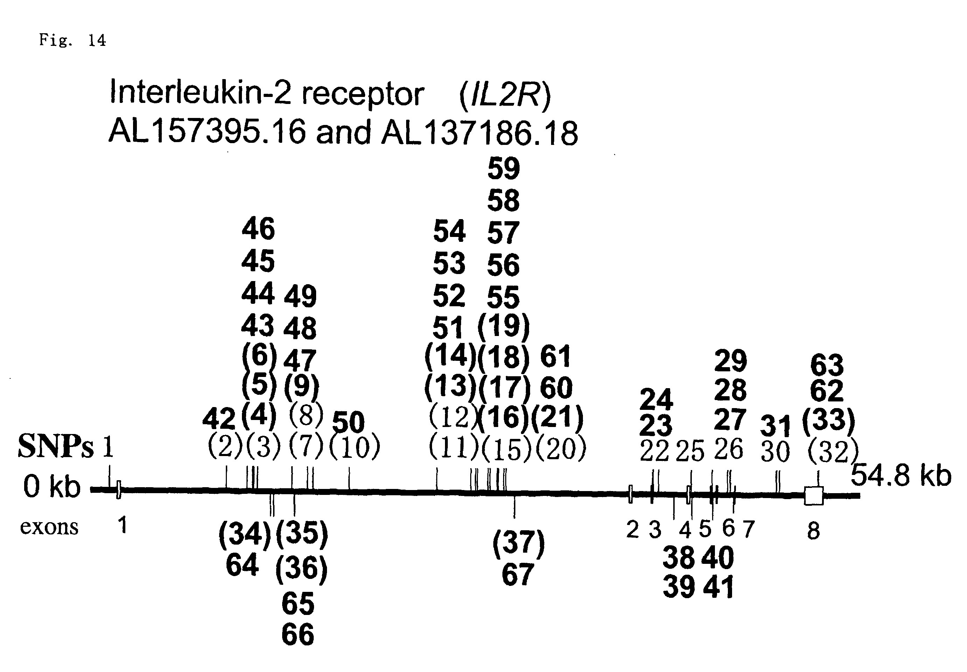

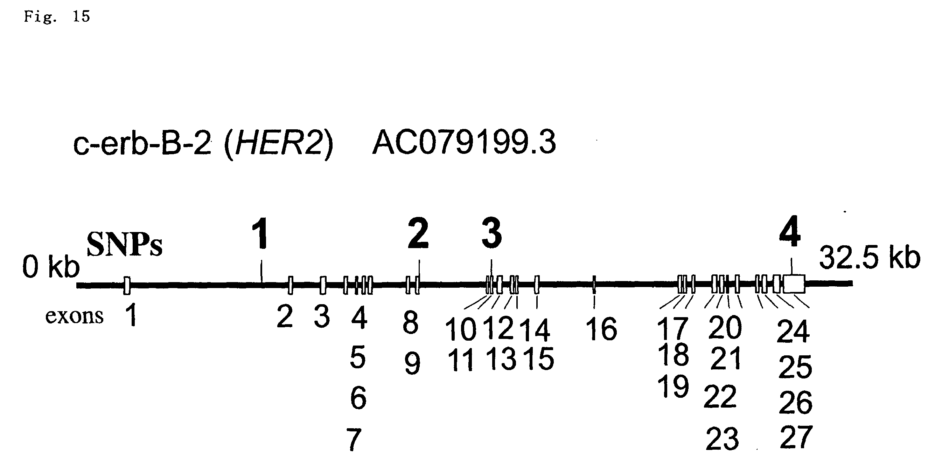

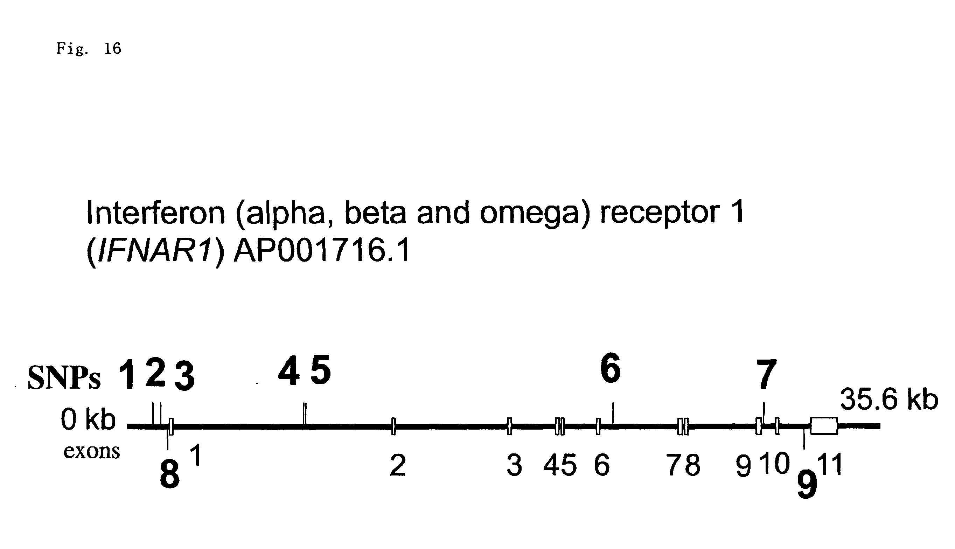

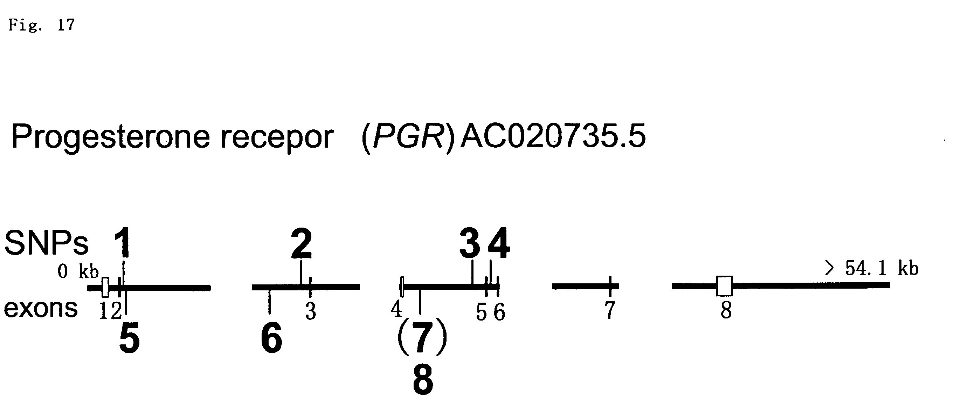

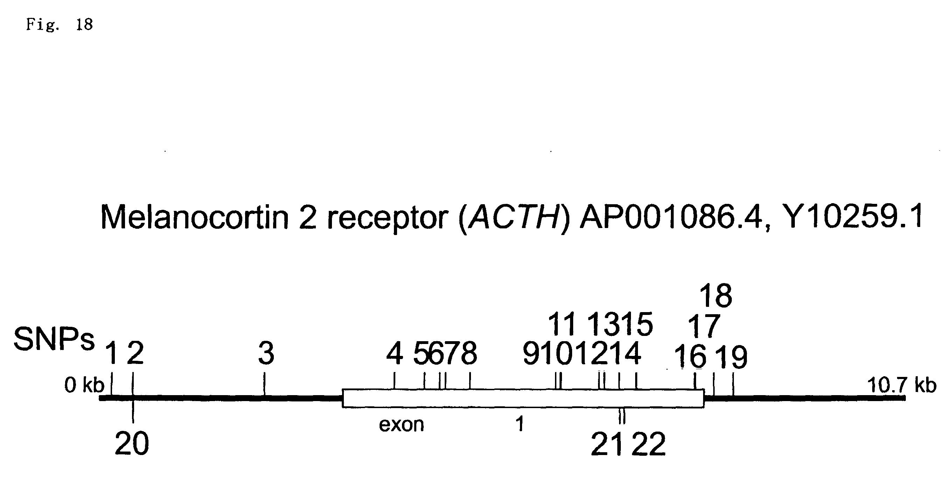

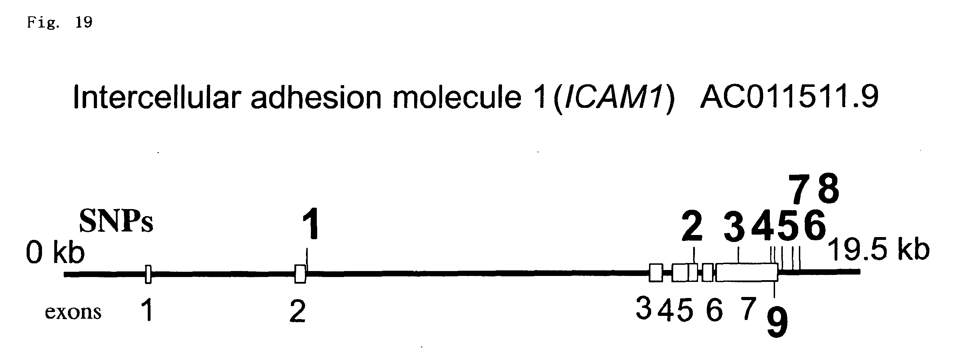

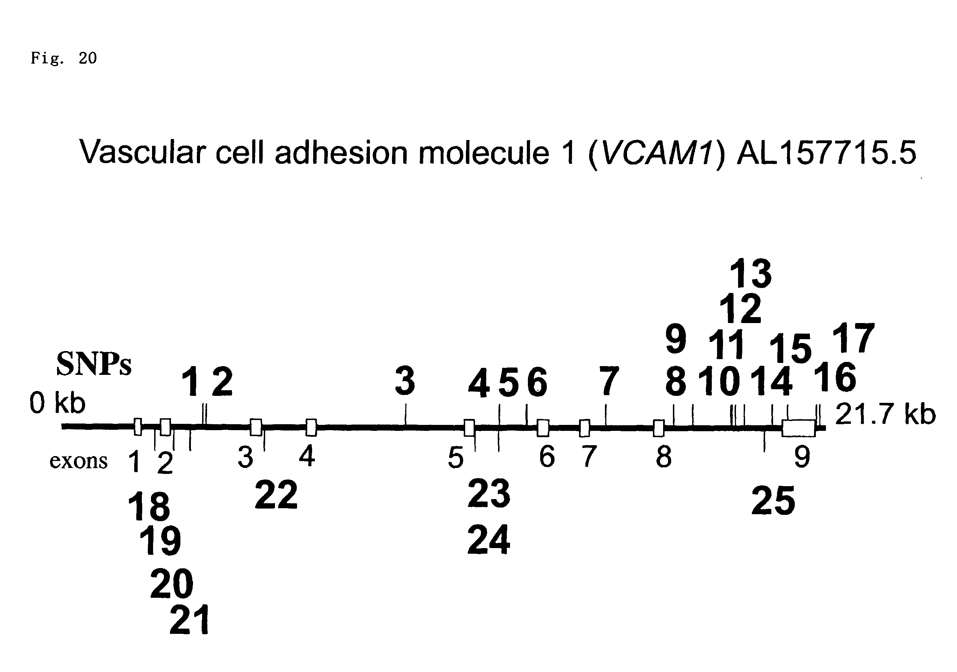

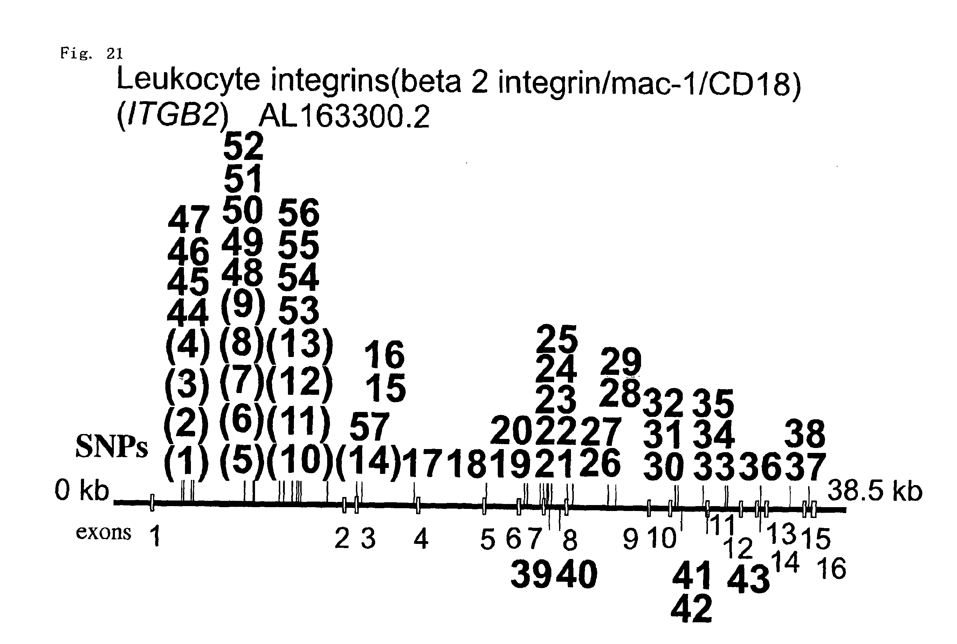

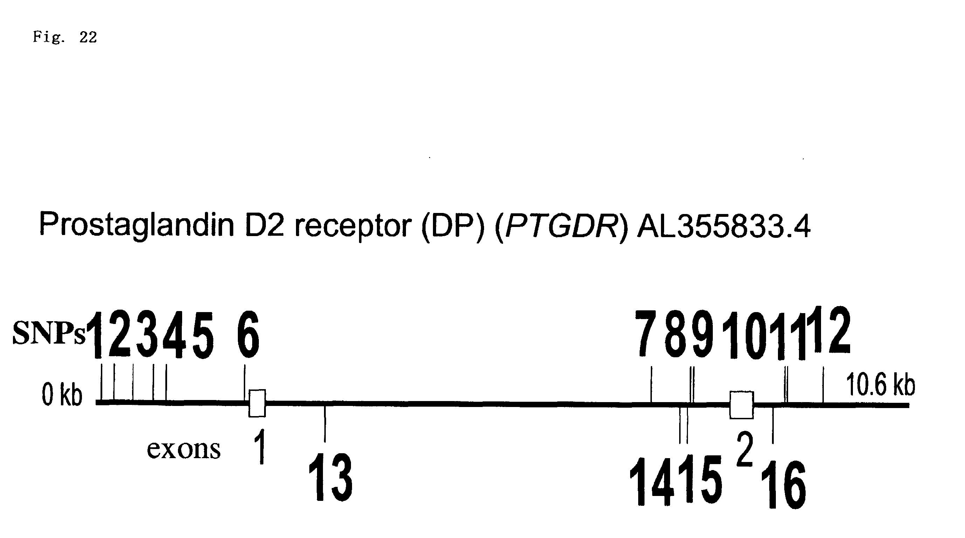

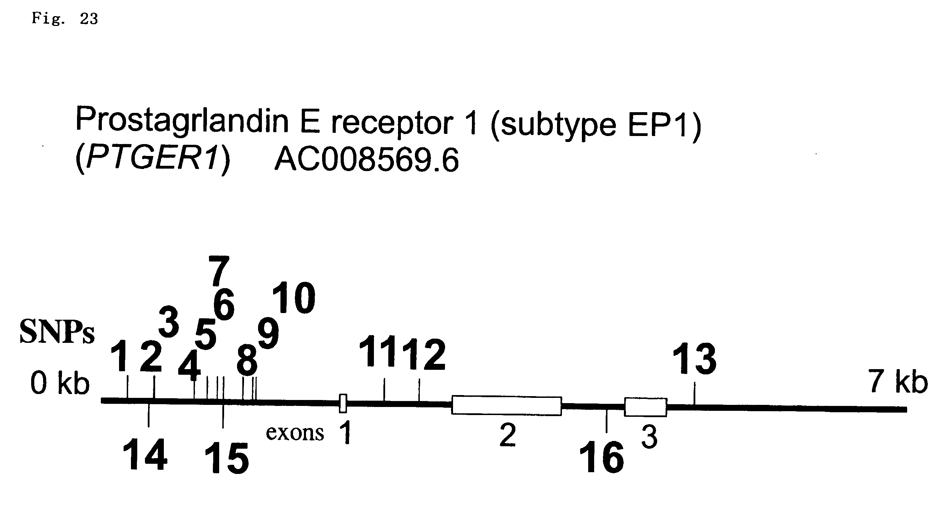

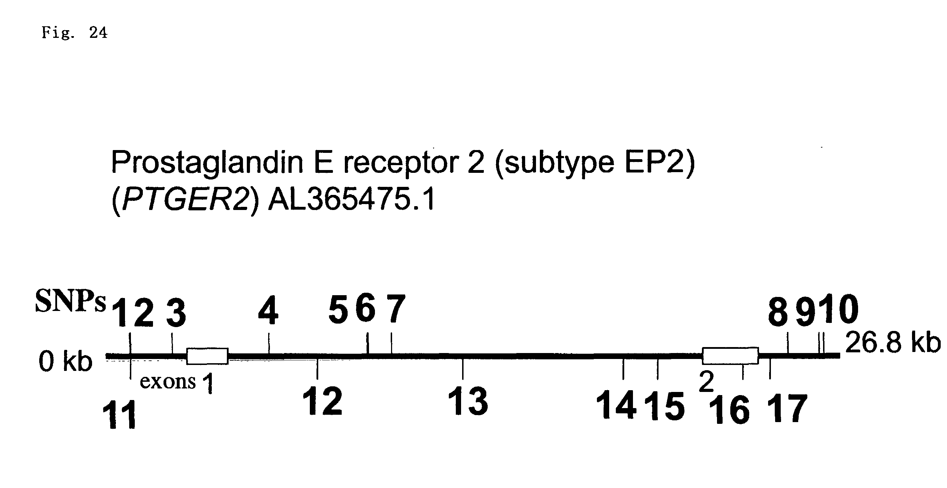

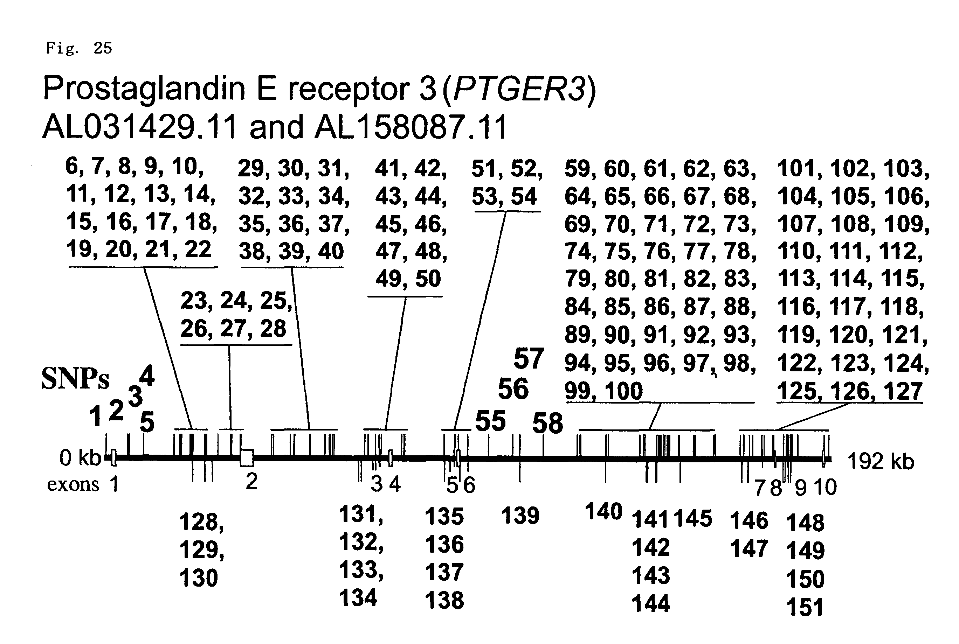

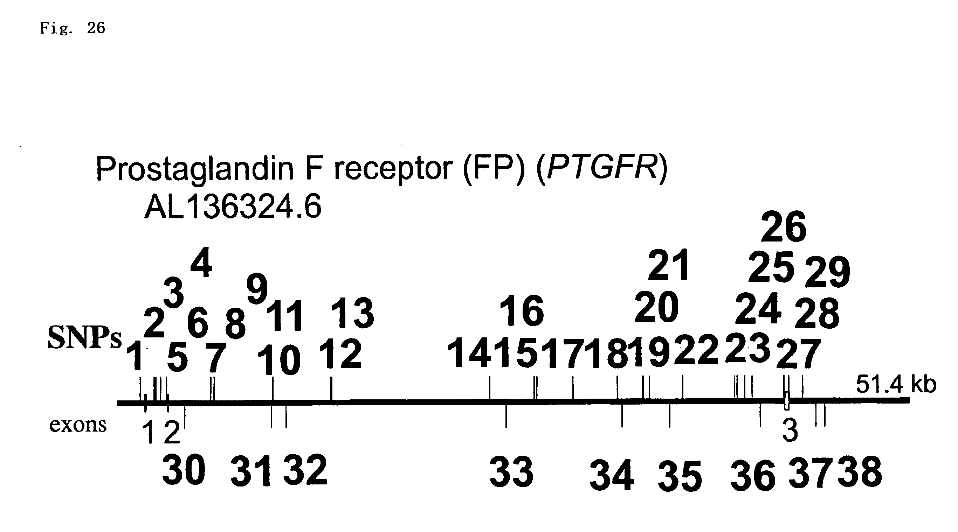

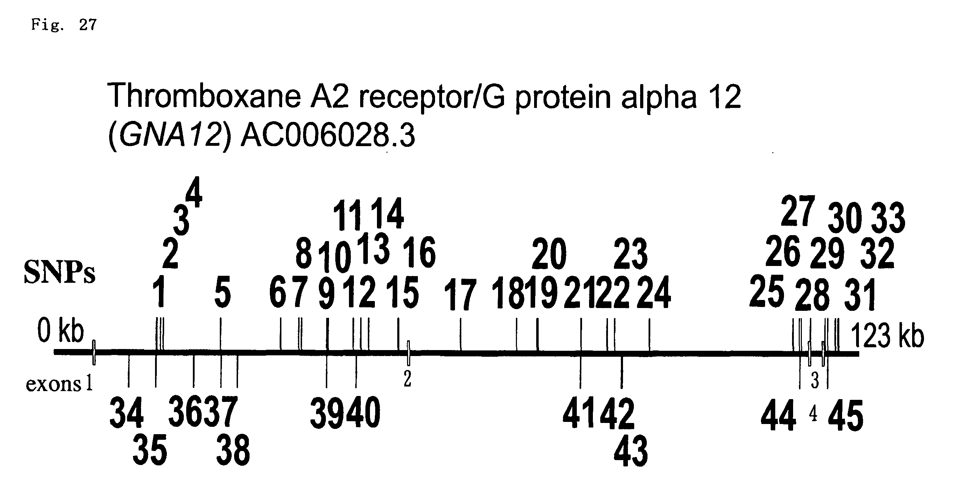

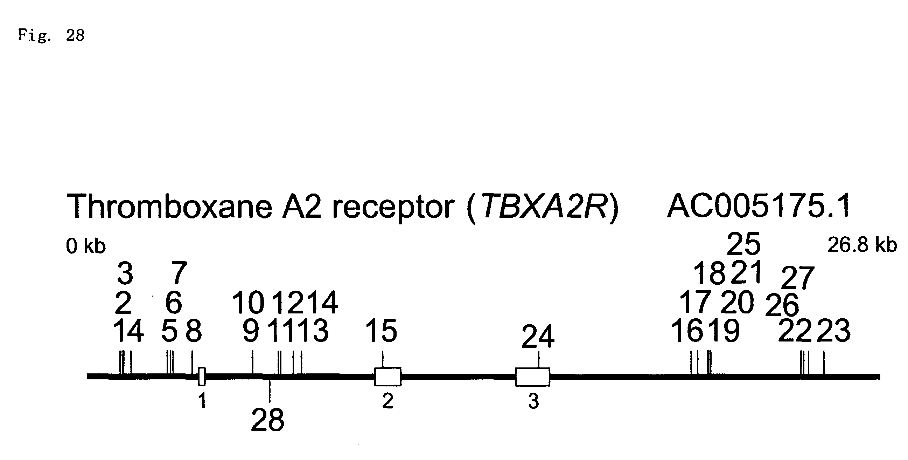

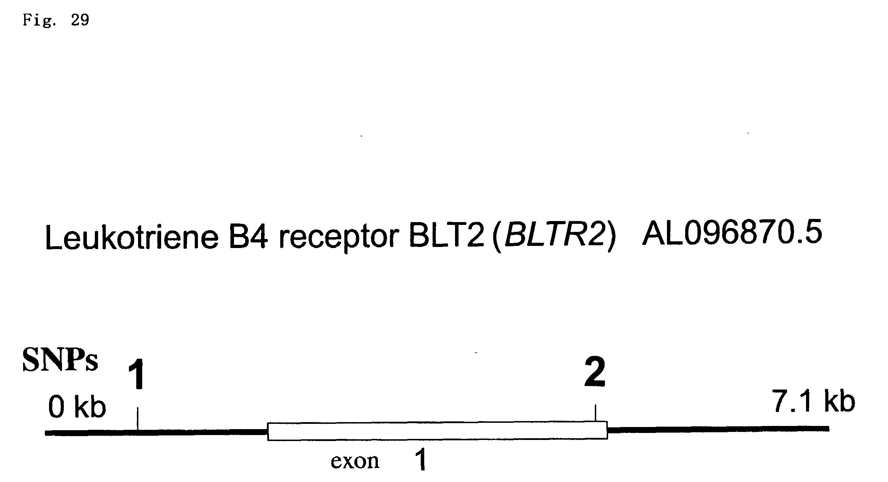

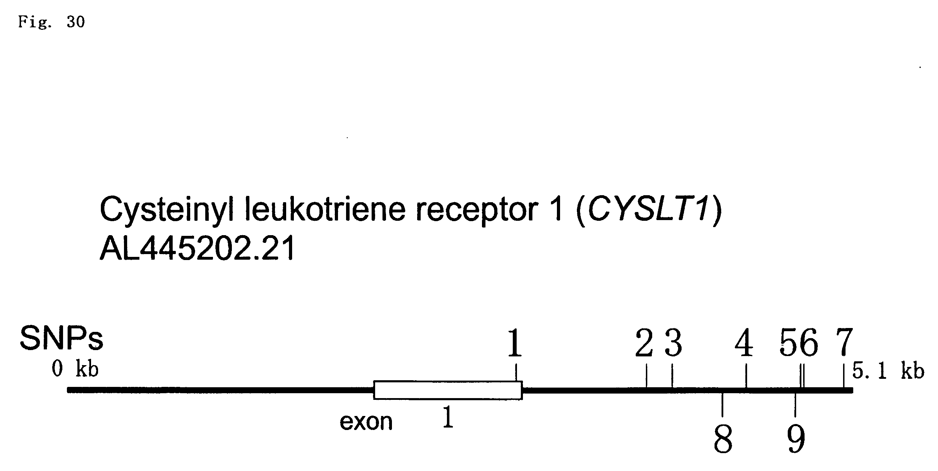

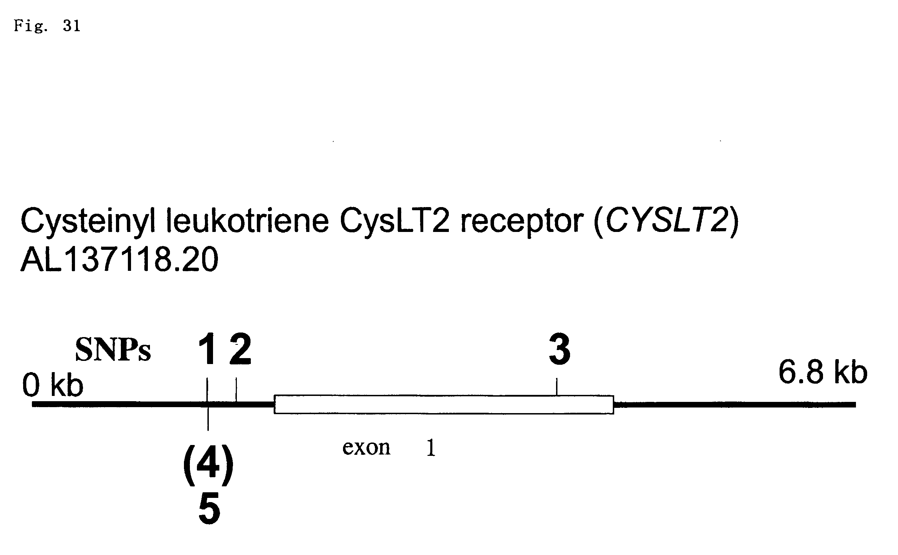

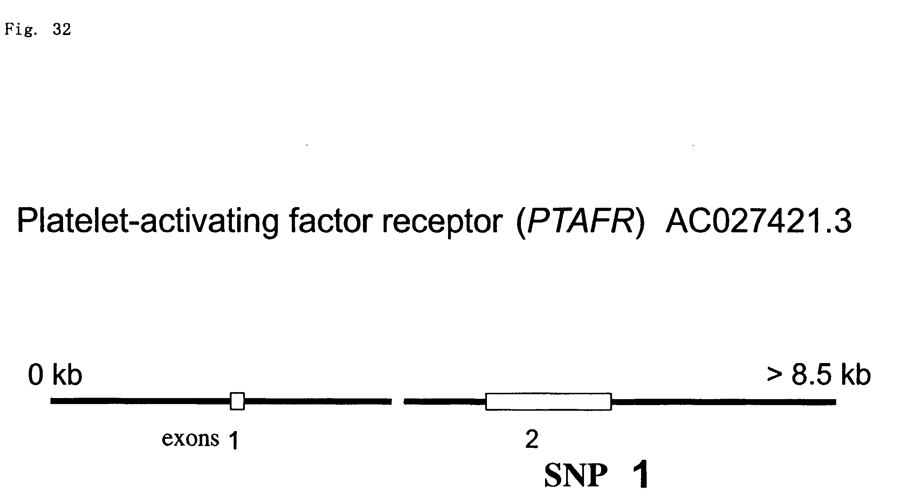

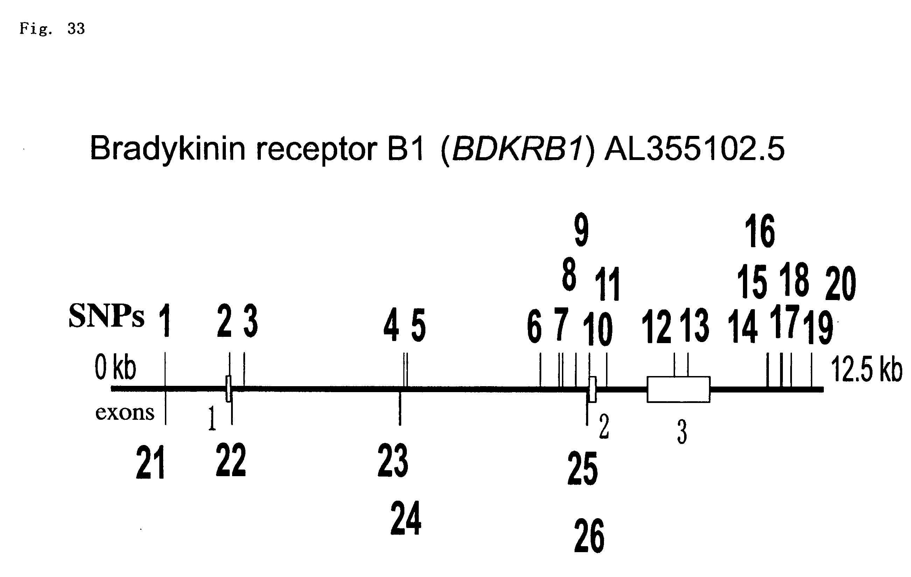

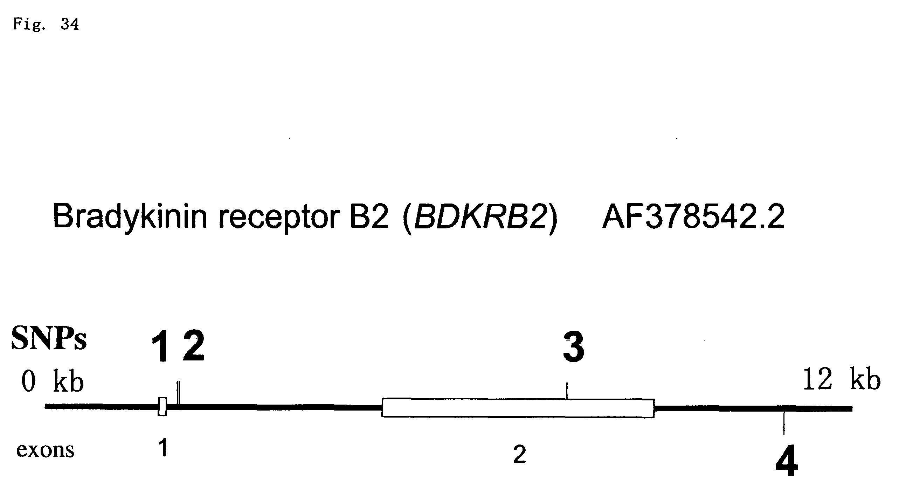

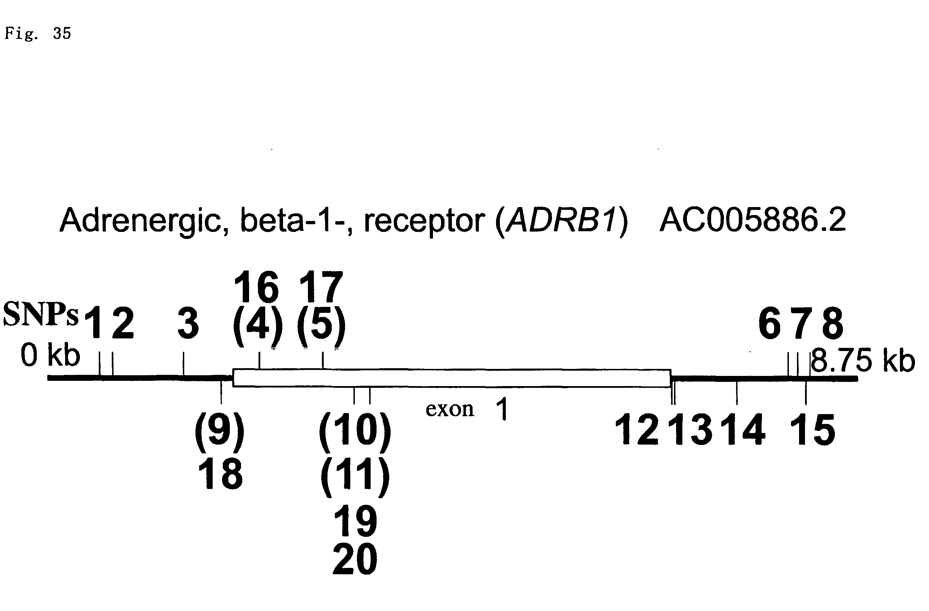

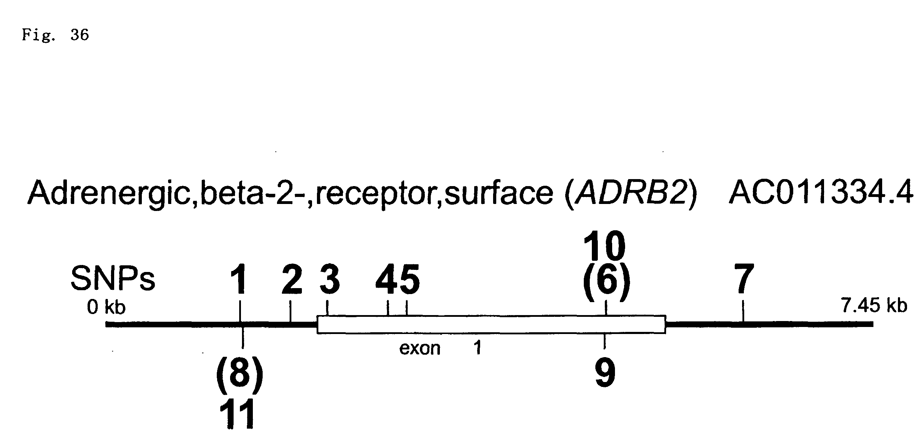

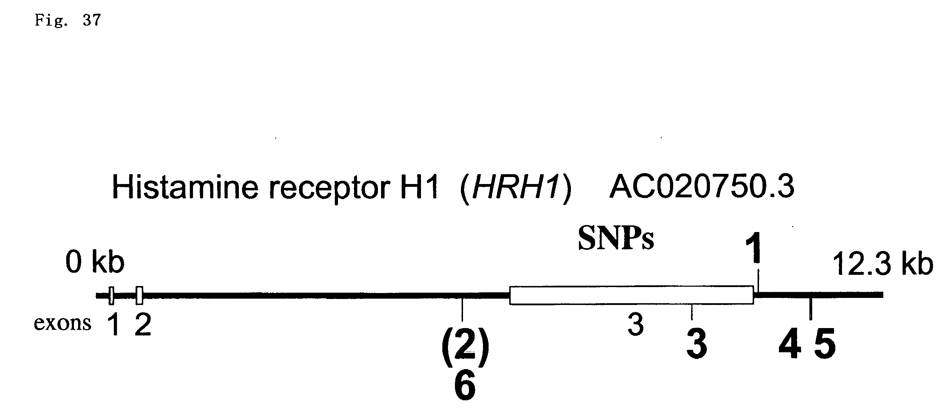

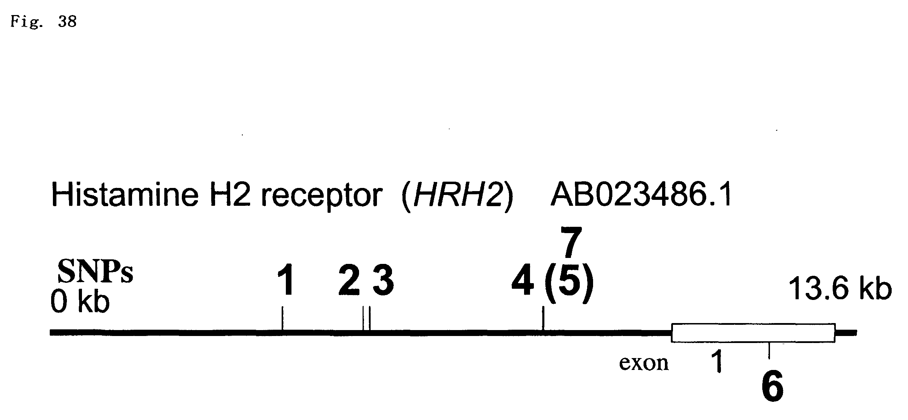

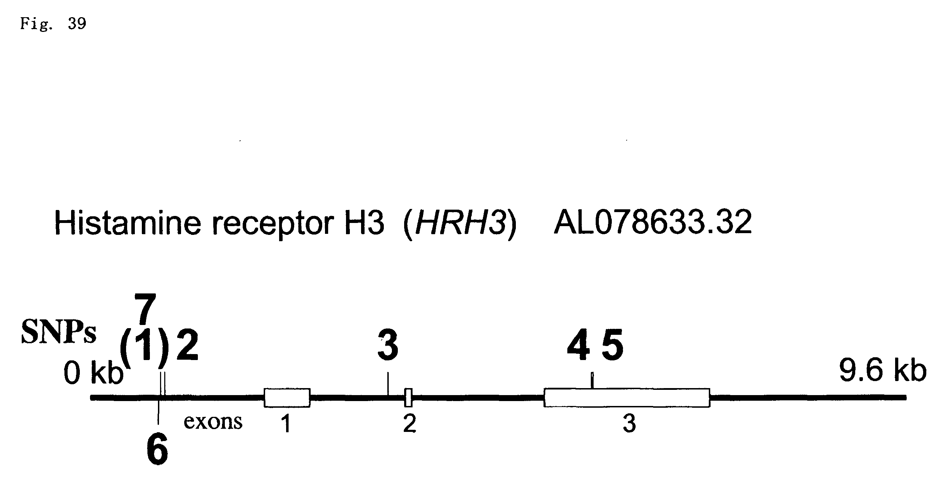

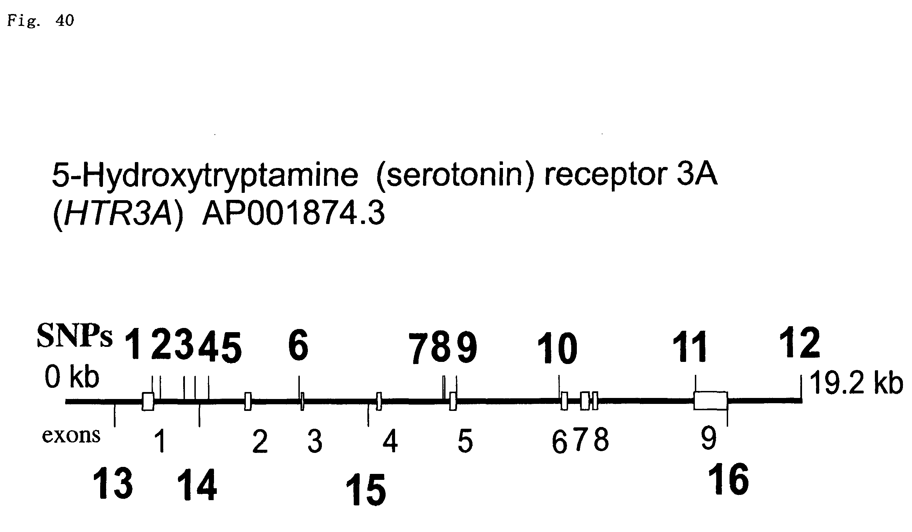

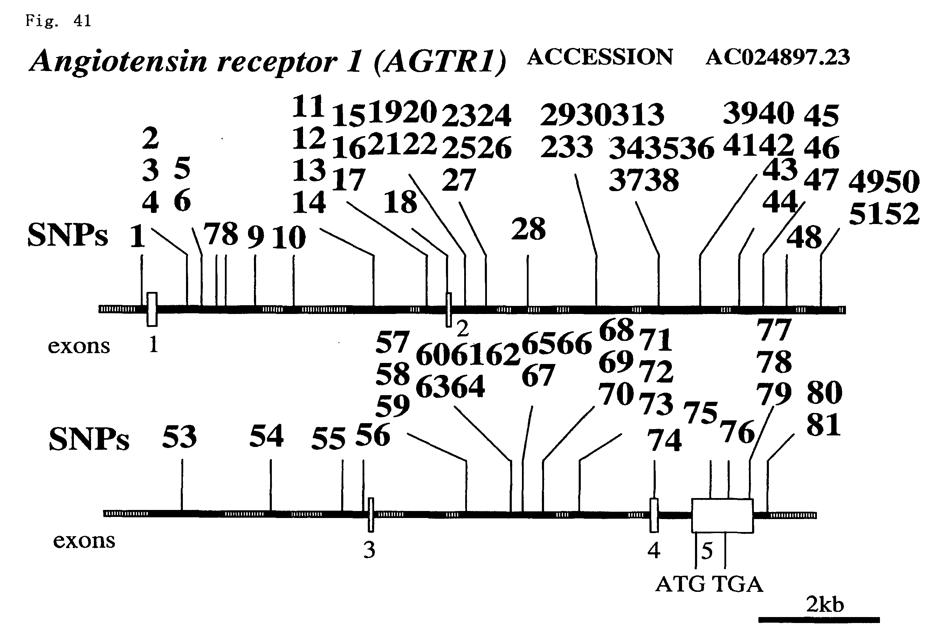

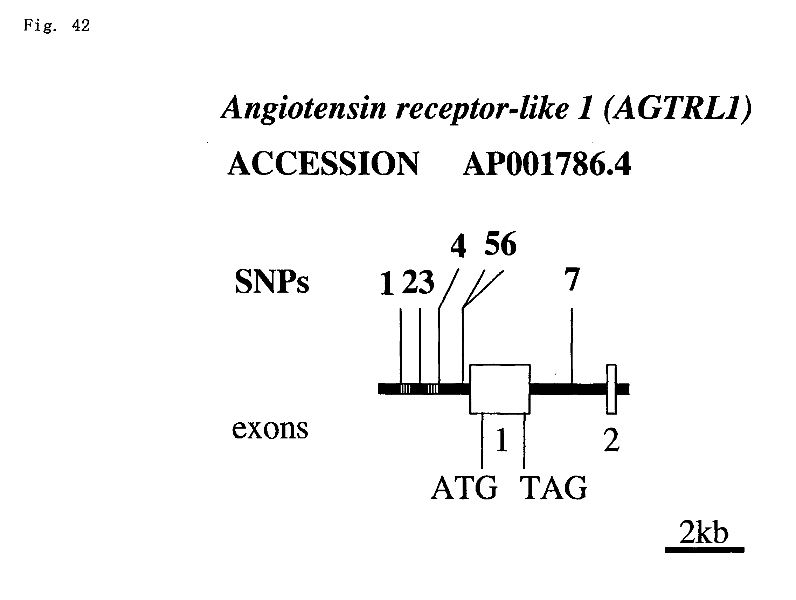

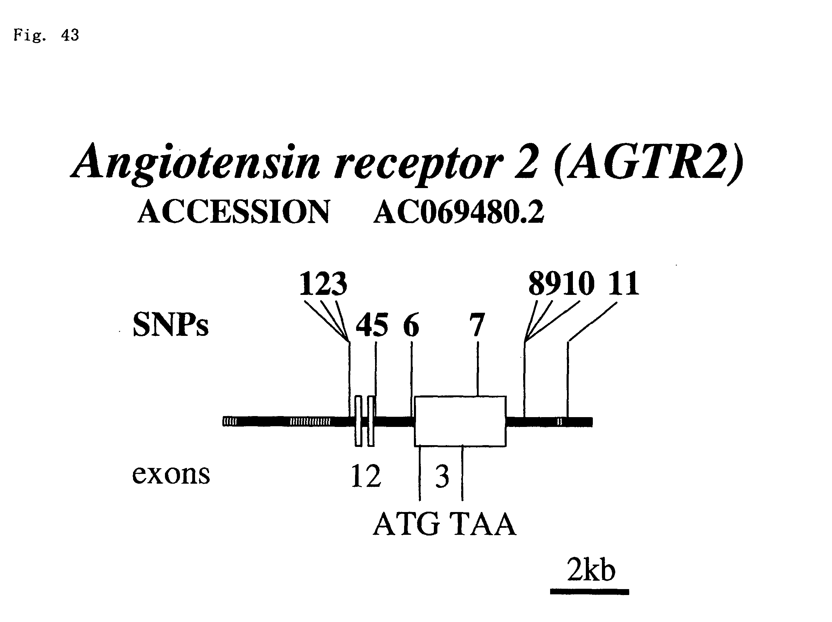

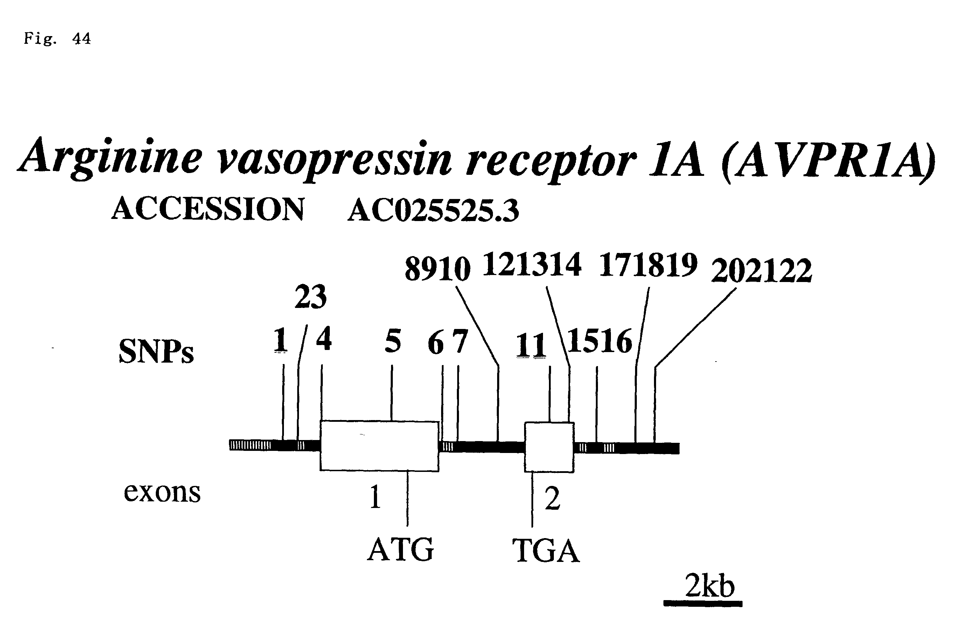

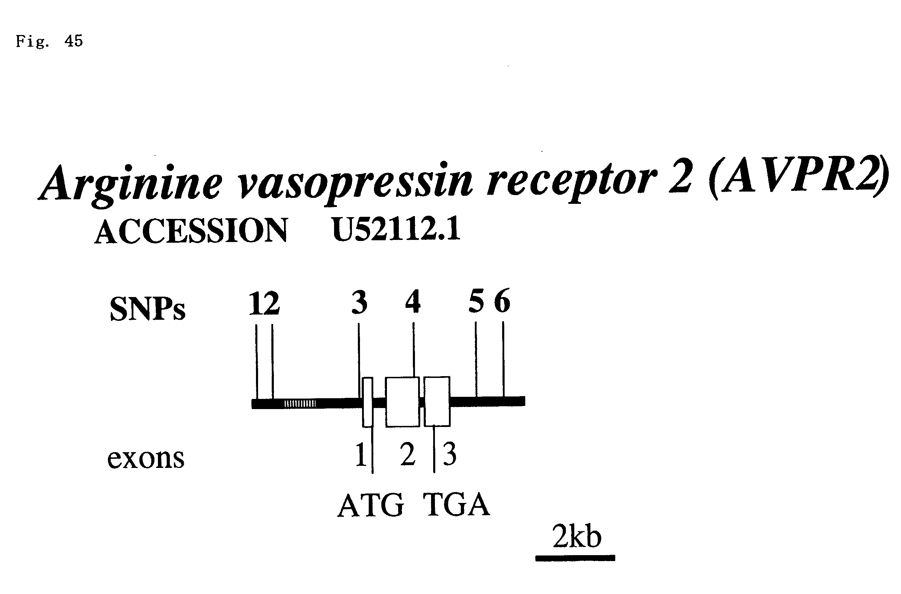

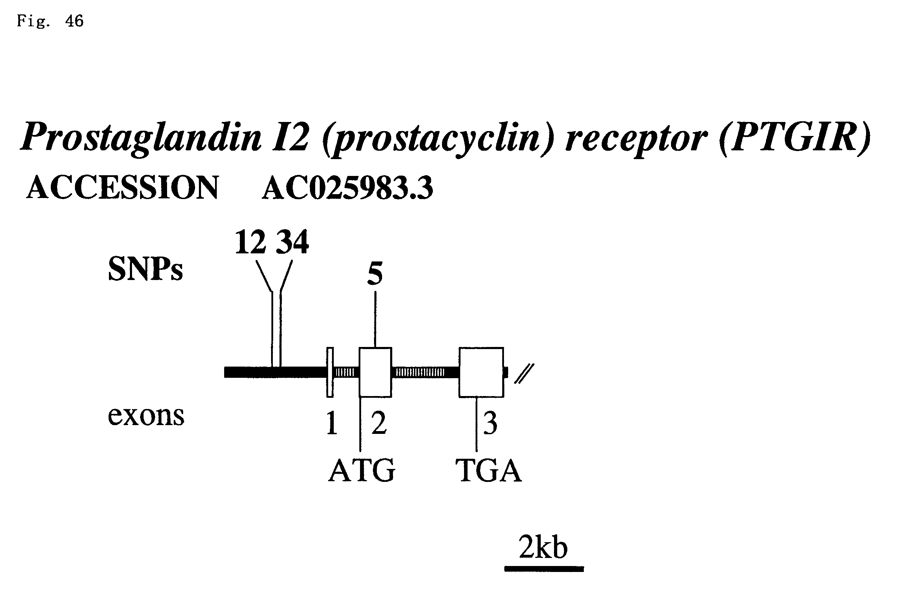

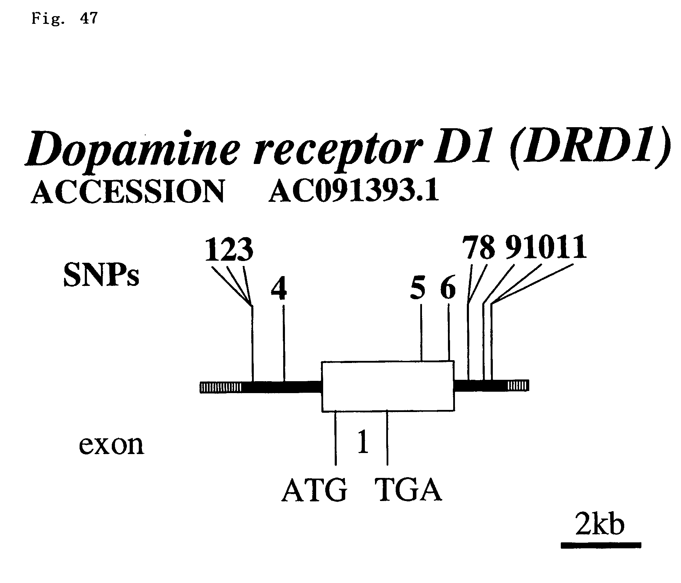

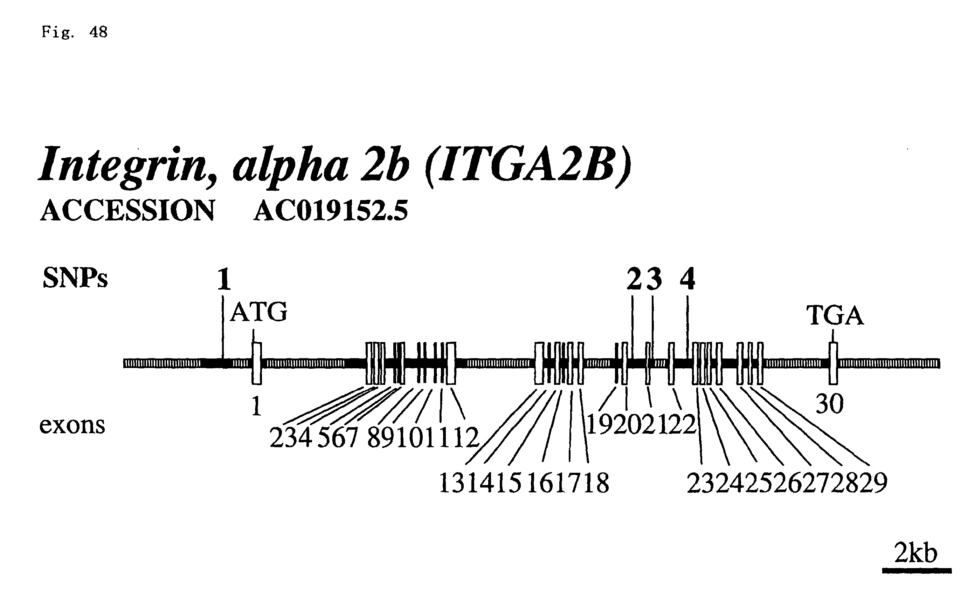

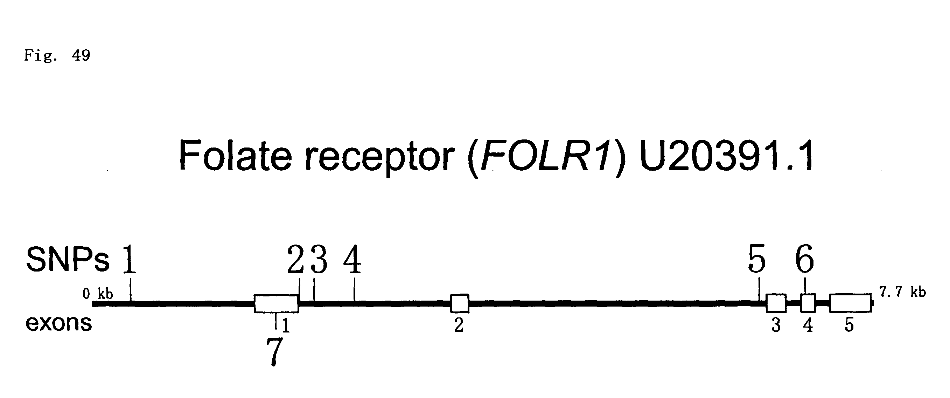

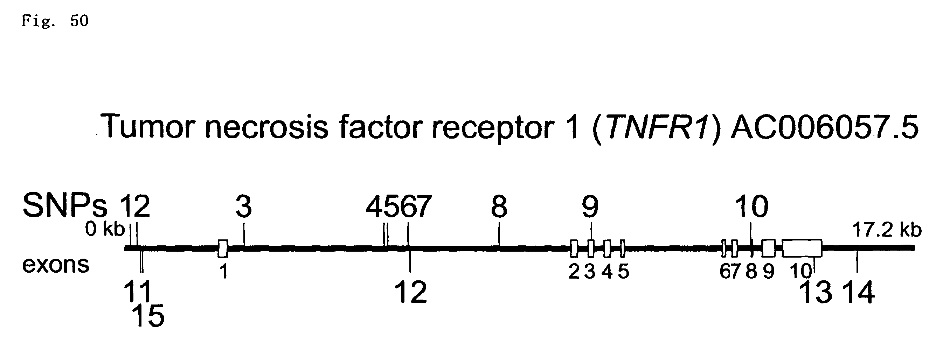

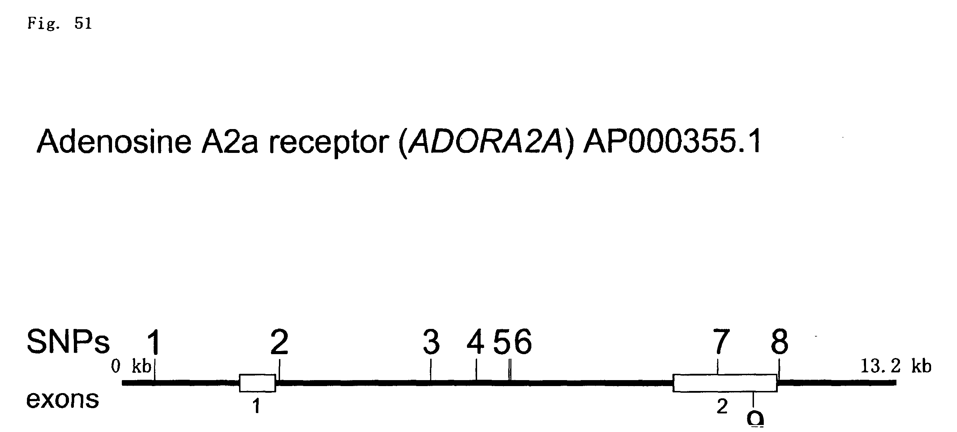

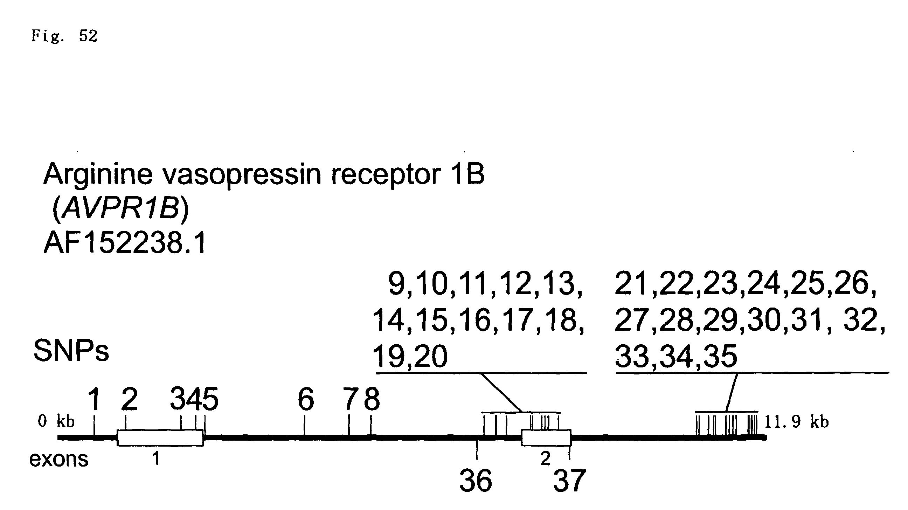

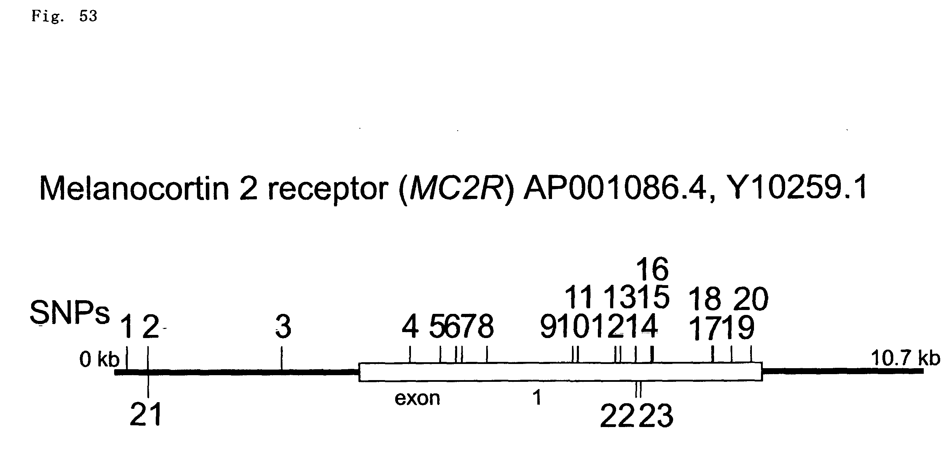

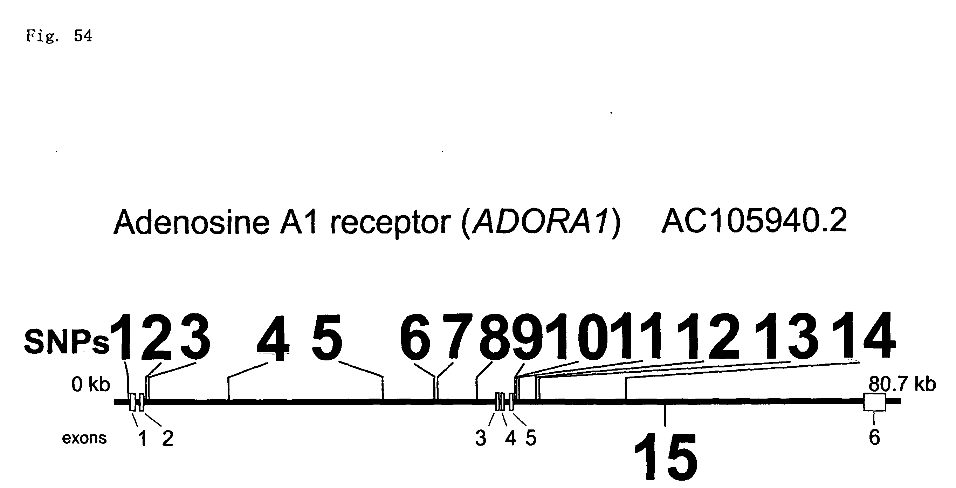

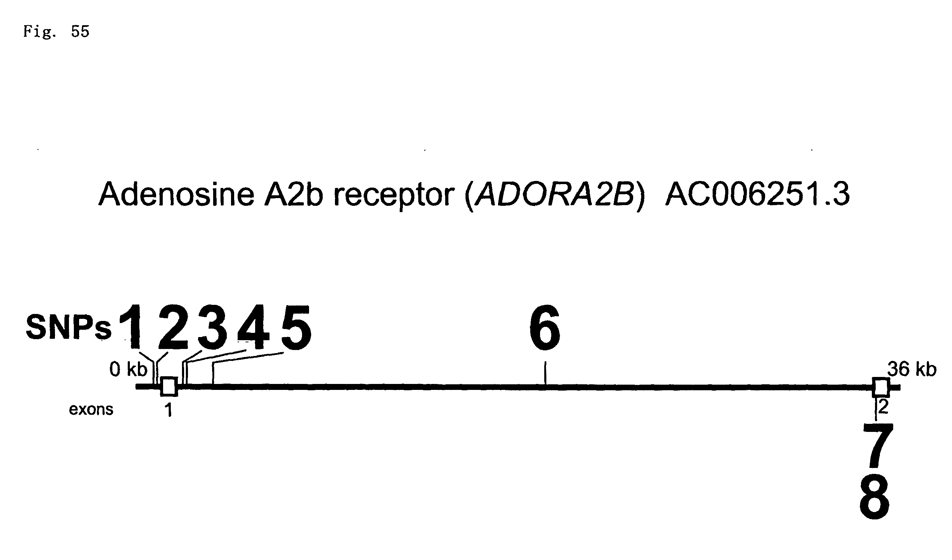

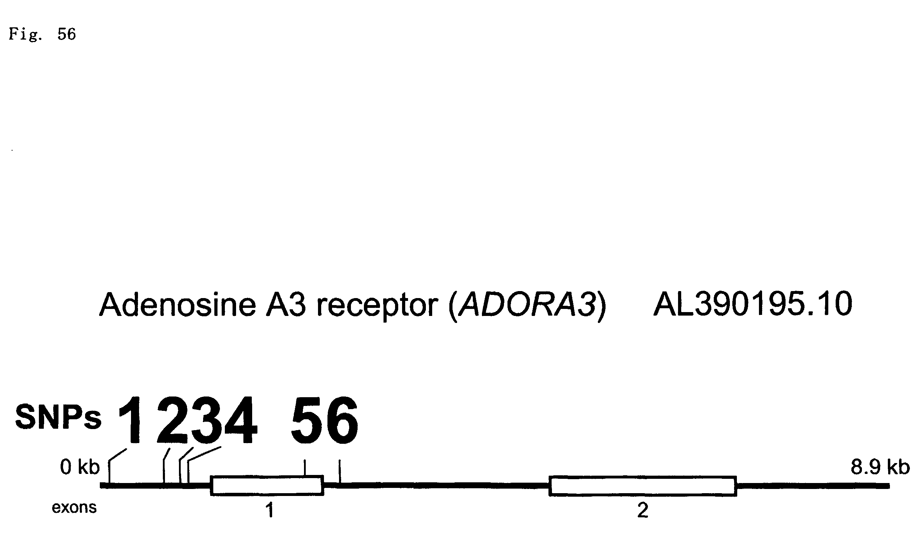

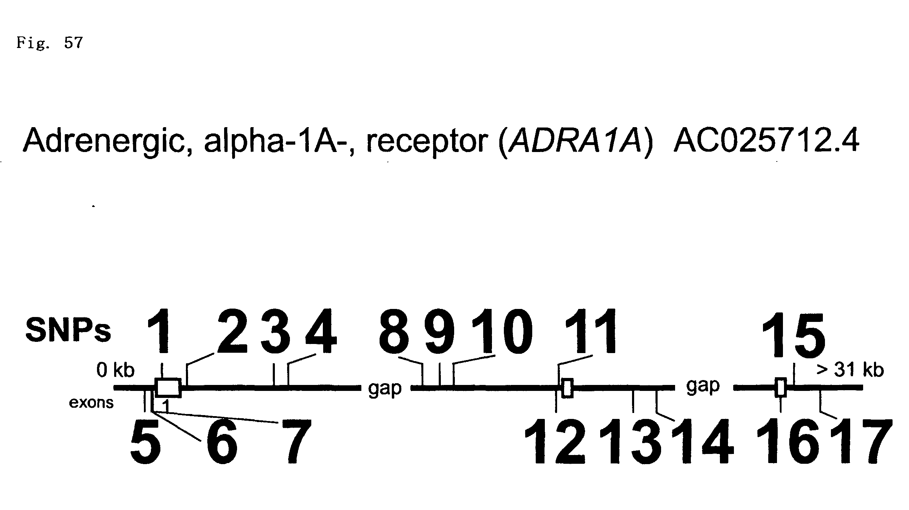

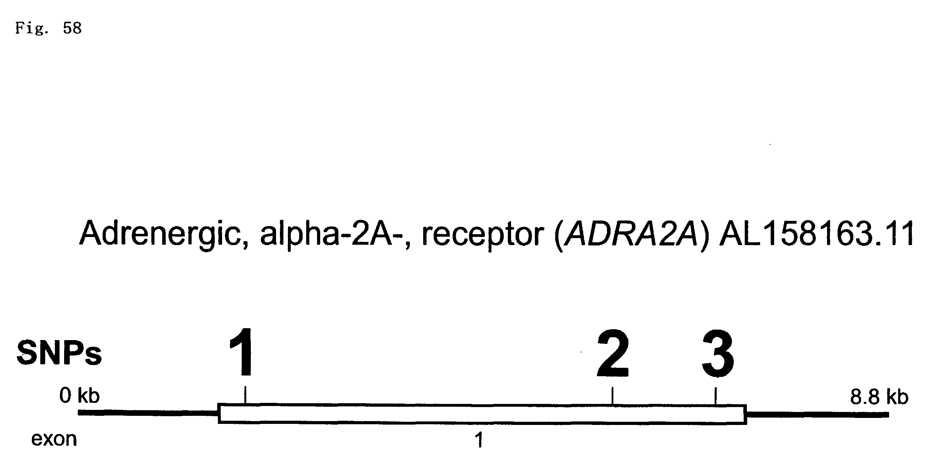

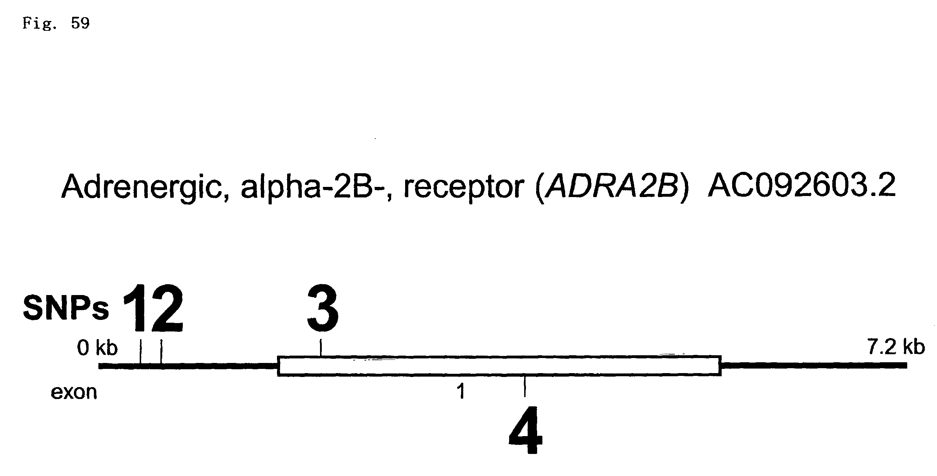

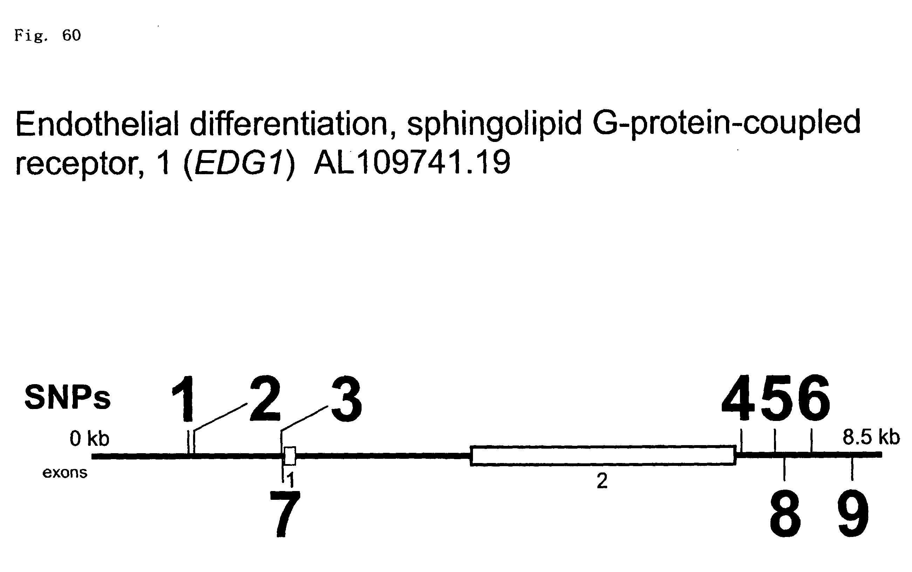

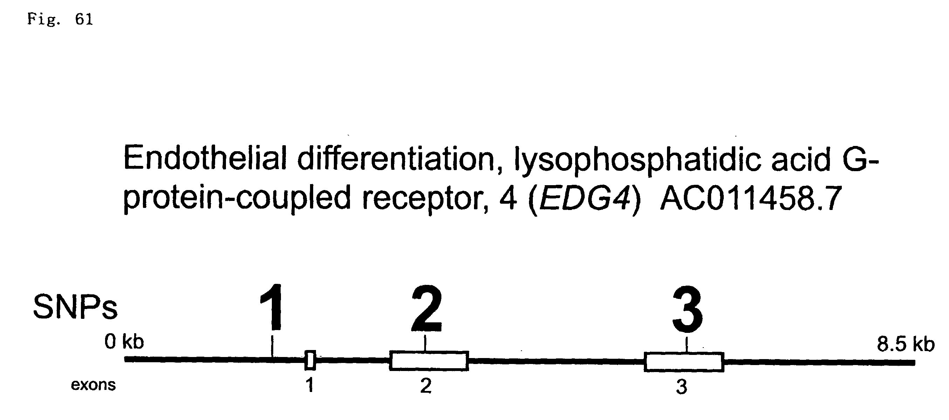

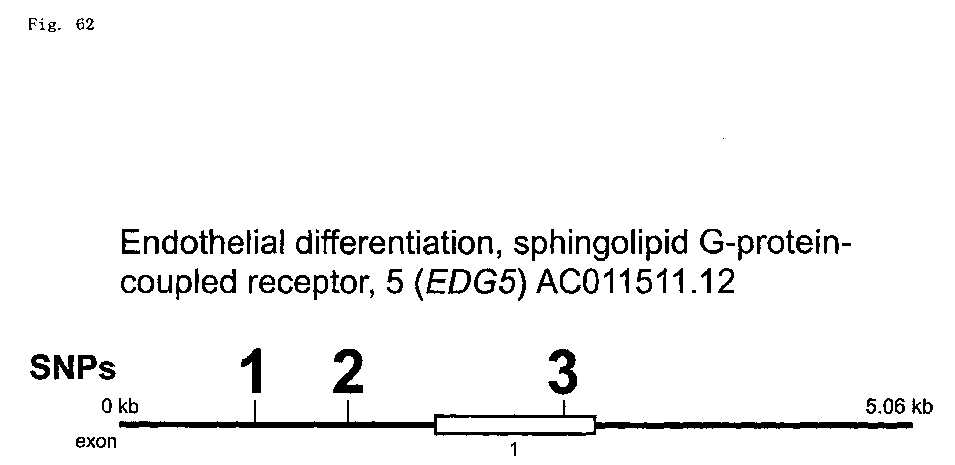

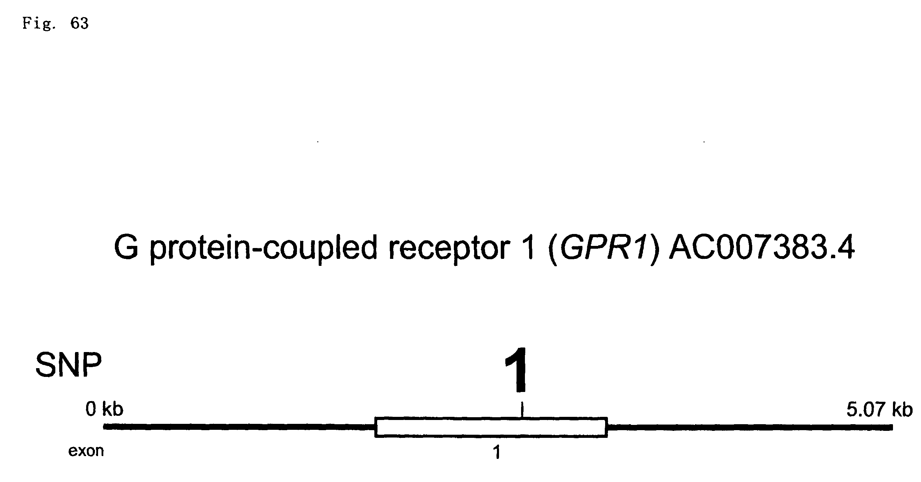

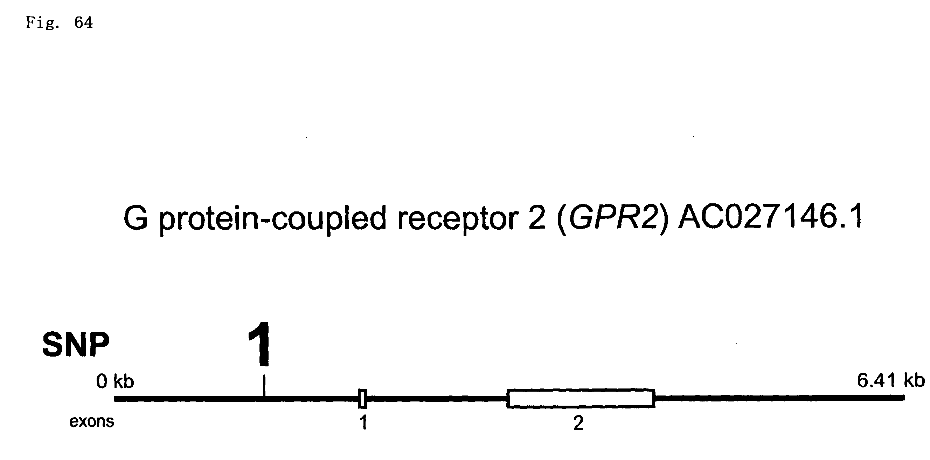

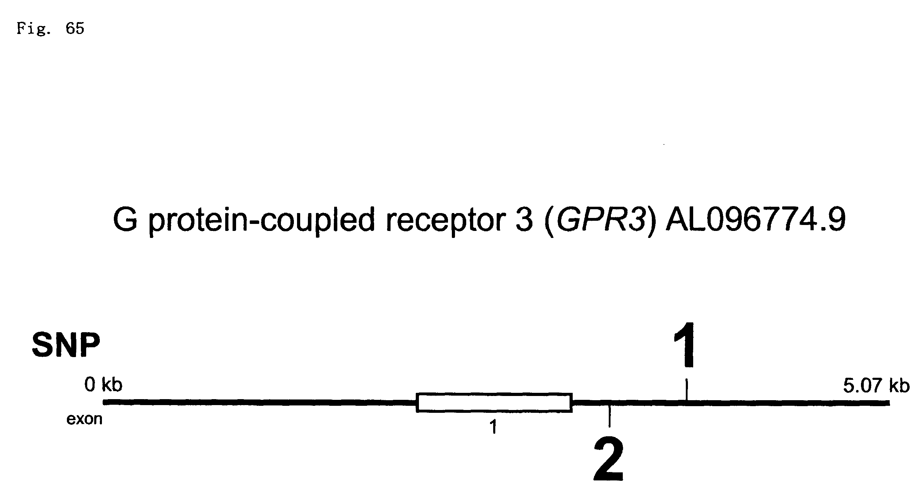

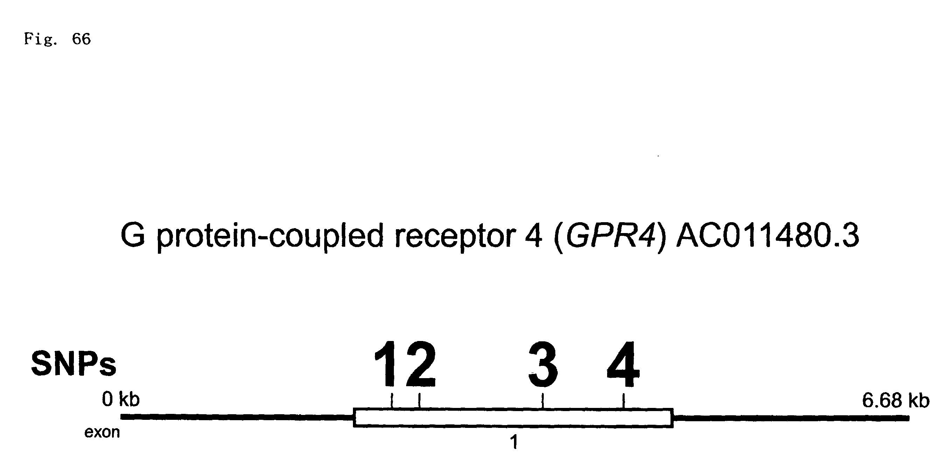

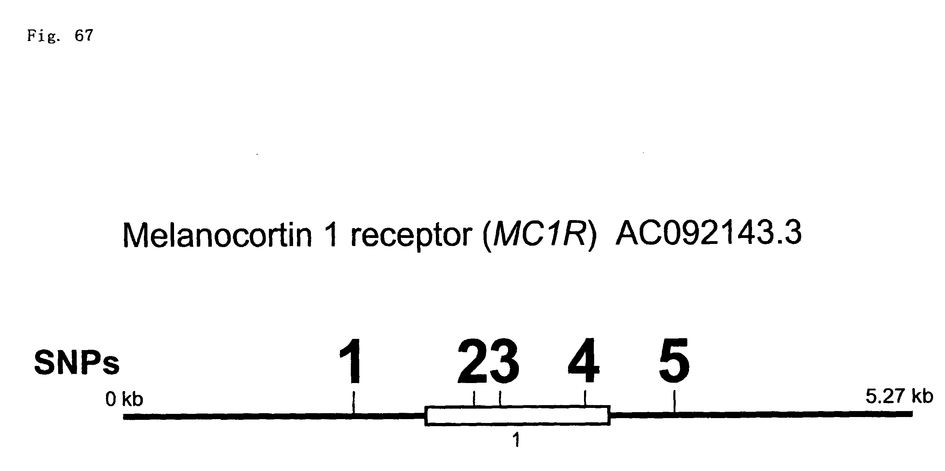

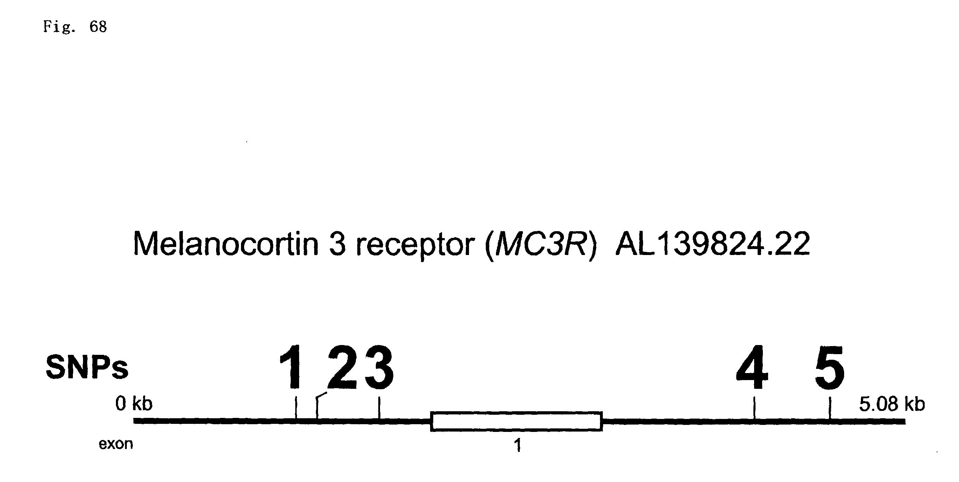

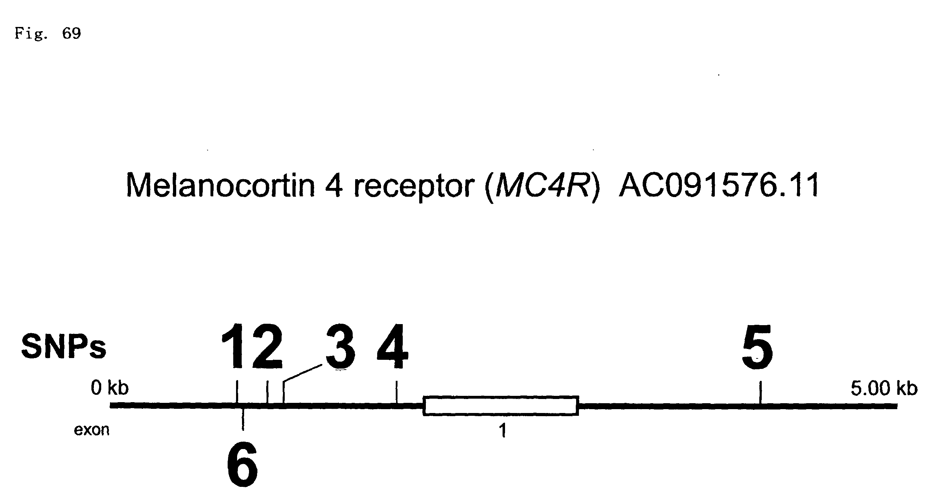

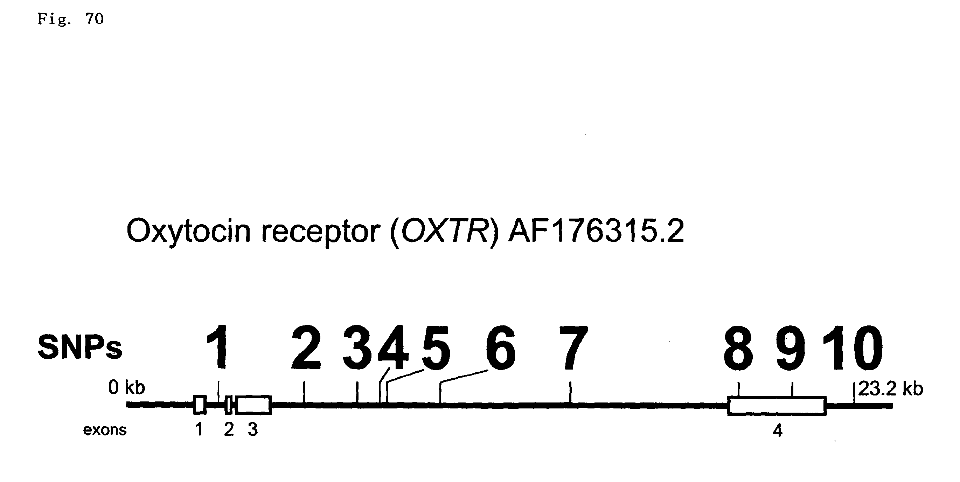

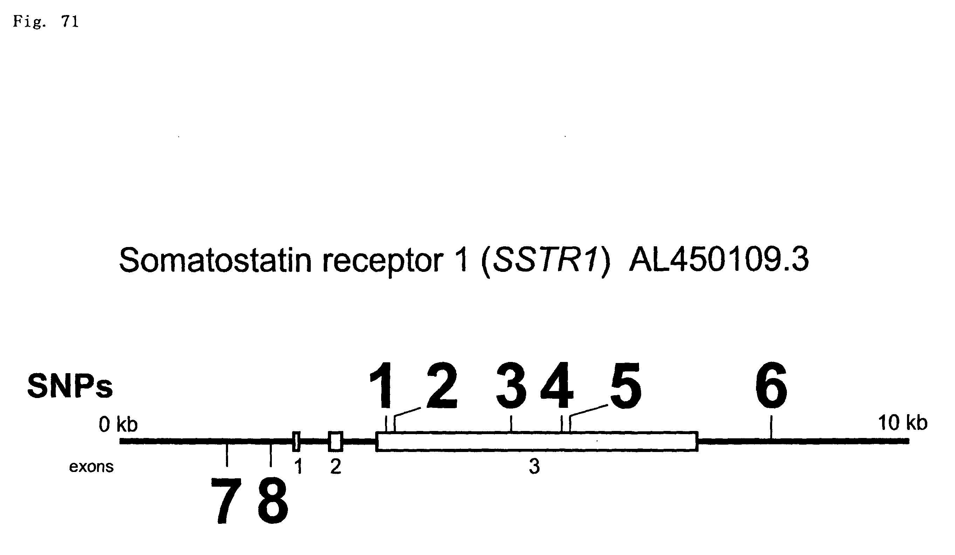

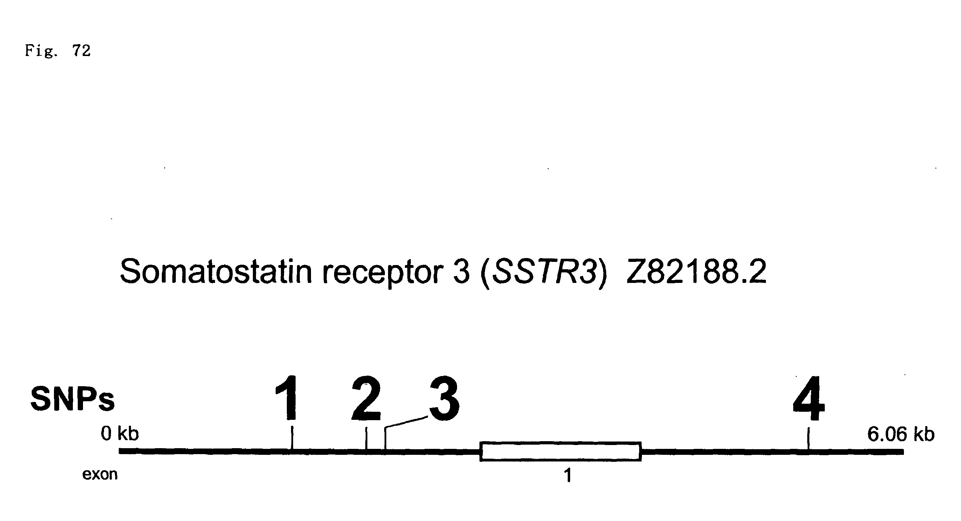

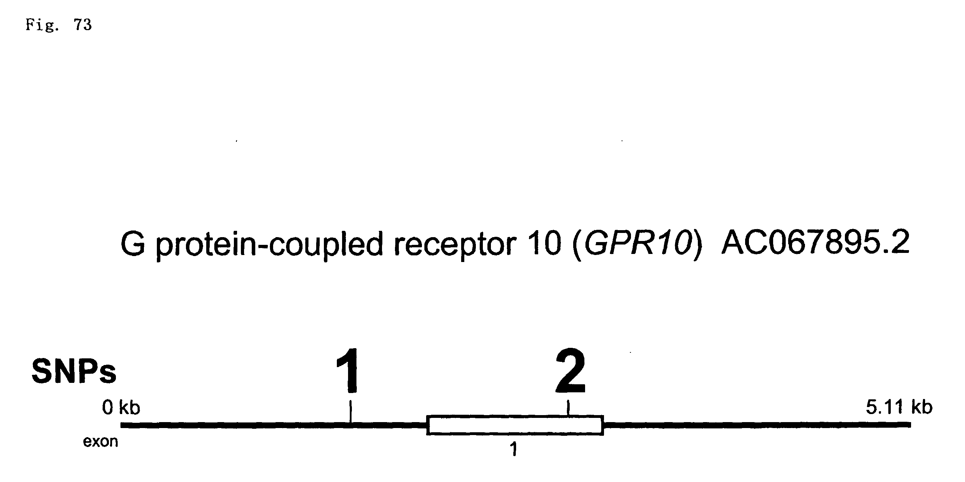

The "Location" shows the location of SNP in the genome. The locations of SNPs in 5' flanking region, intron regions and 3' flanking region are expressed taking the nucleotide located 1 bp upstream of the 5' utmost end nucleotide of exon 1 as -1 position. Then, nucleotides are numbered -2, -3, -4, ... toward the 5' end of the gene. An intron region means a region spanning from a nucleotide positioned 1 bp downstream of the 3' end of an exon to the subsequent exon. The number of an intron is the same as the number of the exon located 5' to the relevant intron. For example, an intron which exists between exon 3 and exon 4 (i.e. the intron located 3' to exon 3) is called intron 3. The locations of SNPs in an intron region are counted taking the first nucleotide located immediately 3' to the exon/intron junction located 5' to the relevant intron as position 1 of the nucleotide sequence of the intron. Numbers with "+" mark or without any mark mean that they are counted toward the 3' end of the gene; numbers with "-" mark mean that they are counted toward the 5' end of the gene. For example, if the third nucleotide counted from exon 3/intron 3 junction toward the 3' end of the gene is polymorphic, the location is expressed as "intron 3, +3". Similarly, if the fifth nucleotide counted from intron 3/exon 4 junction toward the 5' end of the gene is polymorphic, the location is expressed as "intron 3, -5". The region located 3' to the final exon is designated 3' flanking region. The locations of SNPs in this region are counted taking the nucleotide located 1 bp downstream of the 3' utmost end nucleotide of the final exon as position 1. It should be noted that the numbers appearing in the "Location" column in Table 1for MC2R17 - MC2R20 (SEQ ID NOS: 967-970) are those indicated in databases; the numbers obtained by the analysis in the present invention are shown in parentheses. The numbers appearing in the "No." column correspond to the numbers appearing in respective gene maps (Figs. 9-73) which show the locations of SNPs. In Figs. 9-73, accession numbers of polymorphisms in public genome databases are also provided. By using information given in Table 1, Figures and databases, sequences adjacent to polymorphisms can be specified. Such information is useful, for example, in preparing PCR primers adjacent to a polymorphism.

Examples of the above-mentioned genome databases include, but are not limited to, the list of services provided by NCBI (National Center for Biotechnology Information, USA) and IMS-JST JSNP database website (Laboratory for Genotyping, SNP Research Center, RIKEN, Japan).

### 4. Preparation of Oligonucleotide Probes or Oligonucleotide Primers

Oligonucleotides which are used in the detection method of the present invention as primers and/or probes may be prepared based on the nucleotide sequences described in Table 1 (SEQ ID NOS: 1-1168), for example, when SNPs are to be detected, and these sequences *per se* may be synthesized, or primers and/or probes may be designed and synthesized so that they contain a part of these sequences. However, it should be noted here that the nucleotide sequences of such primers or probes must contain an SNP (the portion indicated in capital letters in the "Sequence" column in Table 1). The present invention also includes complementary strands to such sequences.

Taking SNP as an example for the purpose of illustration, a primer or probe is designed so that an SNP site is located at the 3' or 5' end of the nucleotide sequence of the primer or probe; or a primer or probe is designed so that an SNP site is located at the 3' or 5' end of the sequence complementary to its nucleotide sequence; or a primer or probe is designed so that an SNP site is located within four nucleotides, preferably two nucleotides, from the 3' or 5' end of its nucleotide sequence or the sequence complementary thereto. Alternatively, a primer or probe is designed so that an SNP site is located at the center of the full-length nucleotide sequence of the oligonucleotide. The "center" refers to a central region where the number of nucleotides counted from there toward the 5' end and the number of nucleotides counted from there toward the 3' end are almost equal. If the number of nucleotides of the oligonucleotide is an odd number, the "center" is the central five nucleotides, preferably the central three nucleotides, more preferably the single nucleotide at the very center. For example, if the oligonucleotide consists of 41 nucleotides, the "center" is from position 19 to position 23 nucleotides, preferably from position 20 to position 22 nucleotides, more preferably the nucleotide at position 21. If the number of nucleotides of the oligonucleotide is an even number, the "center" refers to the central four nucleotides, preferably the central two nucleotides. For example, if the oligonucleotide consists of 40 nucleotides, the "center" is from position 19 to position 22 nucleotides, preferably the nucleotide at position 20. In the nucleotide sequences shown in the "Sequence" column in Table 1, if the polymorphism is deletion polymorphism, the actual length of such sequences is 40, an even number. Therefore, if an oligonucleotide consisting of 40 nucleotides is designed based on such sequences, the "center" is from position 19 to position 22 nucleotides, preferably the nucleotide at position 20.

When a polymorphic site is composed of a plurality of nucleotides, a probe or primer is designed and prepared so that the entire or a partial nucleotide sequence of the polymorphic site or a sequence complementary thereto is contained in the nucleotide sequence of the probe or primer. When the thus prepared oligonucleotide is used as a probe, it is possible to determine alleles using the presence or absence of hybridization or difference of hybridization. Hereinbelow, those nucleotides in a probe or primer DNA which form a complementary strand with the polymorphic site or its peripheral site are called "corresponding nucleotides". A probe or primer may be designed so that corresponding nucleotides are located on any nucleotide(s) on the sequence constituting a polymorphism. The "peripheral site" means a region one to three nucleotides outside (5' side) of the 5' utmost end of the sequence constituting a polymorphism, or a region one to three nucleotides outside (3' side) of the 3' utmost end of the sequence constituting a polymorphism. In particular, the corresponding nucleotides in a probe or primer can be designed so that the 5' or 3' end nucleotide when forming a complementary strand is located on the 5' terminal side, 3' terminal side, or the center of the sequence constituting a polymorphism. In the present invention, it is preferred that the above-mentioned 5' or 3' end nucleotide is located on the center of the sequence constituting a polymorphism. It is also possible to design corresponding nucleotides so that they are located on a peripheral region of the sequence constituting a polymorphism.

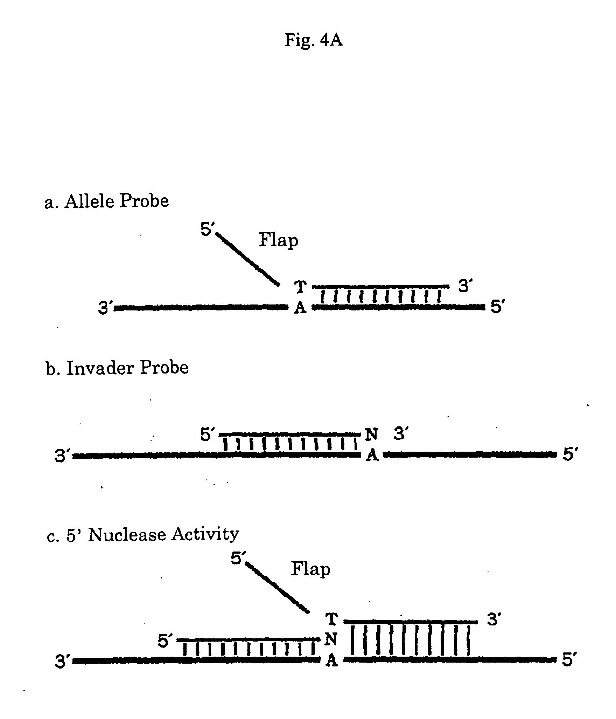

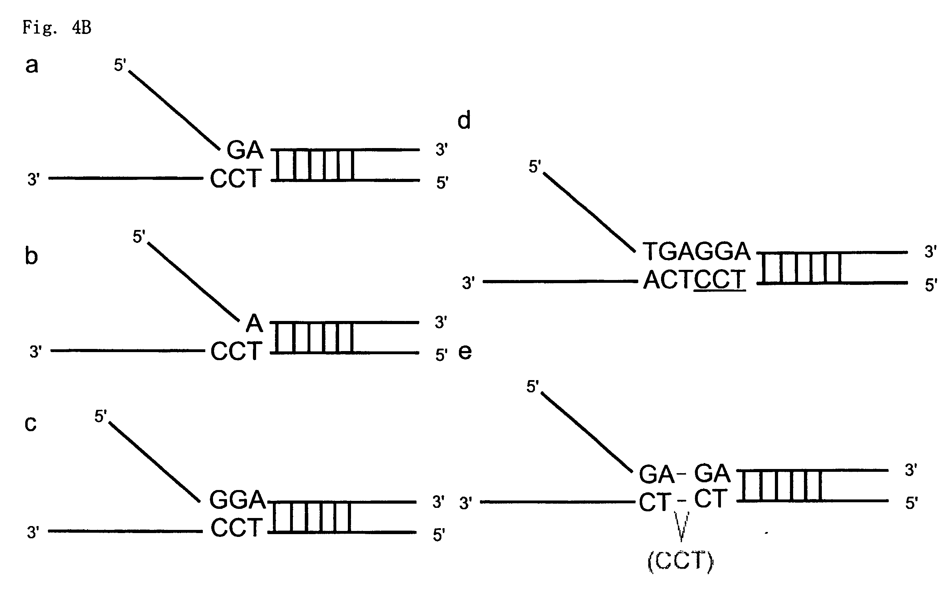

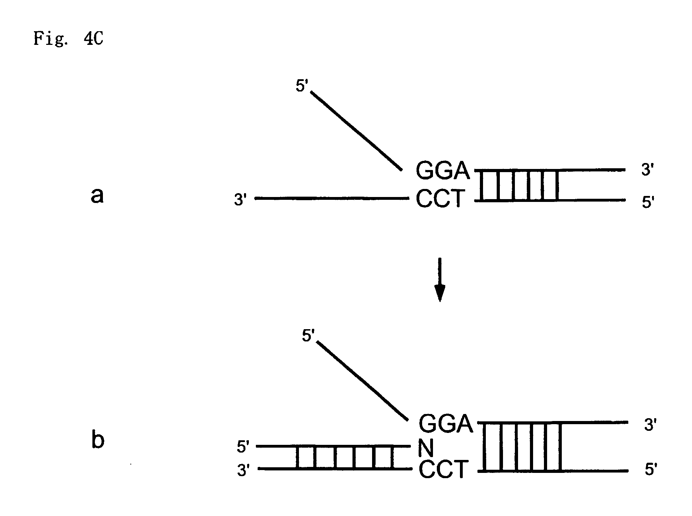

For example, when an invader probe and allele probes are prepared to detect the genetic polymorphism (TCC) as shown in No. 13 of PTGDR (SEQ ID NO: 313) in Table 1 by the invader method described later, allele probes are designed so that nucleotides complementary to the polymorphic site TCC (i.e. G, G or A in the 5' to 3' direction) are the corresponding nucleotides of the allele probes and hybridize (see panels a to c in Fig. 4B). For example, in panel (a) of Fig. 4B, the 5' utmost, corresponding nucleotide "G" forming a complementary strand is located on the center of the nucleotides (TCC) constituting a polymorphism; in panel (b) of Fig. 4B, the 5' utmost, corresponding nucleotide "A" forming a complementary strand is located on "T" of the nucleotides (TCC) constituting a polymorphism. In panel (d) of Fig. 4B, an allele probe is shown which is designed so that its corresponding nucleotides are located on the peripheral region (three nucleotides) of the polymorphic site (the underlined portion).

An invader probe is designed so that the position of its 3' end nucleotide "N" (any one of A, T, C or G) corresponds to the position of any of the nucleotides of the polymorphic site (TCC) when hybridized. However, it is most effective to design an invader probe so that there is an overlap of one nucleotide between the invader probe and the allele probe (Fig. 4C). On the other hand, in order to detect another polymorphism (i.e. deletion of TCC), the invader probe and allele probe may be designed so that a nucleotide located one to three nucleotides 5' side or 3' side of the deletion site will be the overlapping nucleotide taking into consideration of the deletion of TCC (i.e. deleting from the sequence). Panel (e) of Fig. 4B shows an allele probe which is designed so that the two nucleotides of the both sides of the deletion site hybridize thereto. With respect to TaqMan probes, they may be designed so that any of these nucleotides TCC or the deletion site is located at the center of the probe.

The length of the nucleotide sequence is designed so that at least 13 nucleotides, preferably 13 to 60 nucleotides, more preferably 15 to 40 nucleotides, and most preferably 18-30 nucleotides are contained. This oligonucleotide sequence may be used as a probe for detecting a target gene, and it may be used as either a forward (sense) primer or a reverse (antisense) primer.

The oligonucleotide used in the invention may be an oligonucleotide composed of two regions connected in tandem, one region being hybridizable to the genomic DNA and the other region being not hybridizable thereto. The order of connection is not particularly limited; either region may be located upstream or downstream. The hybridizable region of this oligonucleotide is designed based on the information on SNP-containing sequences described in Table 1. The oligonucleotide is prepared so that the nucleotide located at the 5' or 3' utmost end of the region hybridizable to the genomic DNA corresponds to an SNP of interest. The region of the above oligonucleotide not hybridizable to the genomic DNA is designed at random so that it does not hybridize to the SNP-containing sequence described in Table 1. This oligonucleotide may be used as a probe mainly for detecting SNPs in the invader method.

Further, the primer used in the present invention is designed so that a nucleotide sequence given in Table 1 contains an SNP when amplified by PCR for the purposes of examining functional changes resulted from the SNP, judging the efficacy or non-efficacy, and examining the occurrence of side effect. The length of the primer is designed so that at least 15 nucleotides, preferably 15 to 30 nucleotides, more preferably 18 to 24 nucleotides are contained in the primer. The primer sequence is appropriately selected from the template DNA so that the amplified fragment has a length of 1000 bp or less, preferably within 500 bp (e.g. 120 to 500 bp), more preferably within 200 bp (120 to 200 bp).

For example, primers are designed so that at least one of the sense or the antisense primer is contained in a region within 1000 bp, preferably 200 bp, more preferably 100 bp counted from the nucleotide immediately adjacent to 5' or 3' to the SNP toward the 5' or 3' end of the gene, respectively.

The thus designed oligonucleotide primers or probes may be synthesized chemically according to known techniques. Usually, such primers or probes are synthesized with a commercial chemical synthesizer.

It is also possible to label probes with fluorescent substances (e.g. FAM, VIC, Redmond Dye, etc.) in advance to thereby automate detection procedures.

### 5. Kit

The above-described oligonucleotide may be included in a genetic polymorphism detection kit. The genetic polymorphism detection kit of the present invention comprises one or more components necessary for practicing the present invention. For example, the detection kit of the present invention comprises components to preserve or supply enzymes and/or reaction components necessary for performing cleavage assay (e.g. invader assay). The kit of the present invention comprises any and all components enzymes or components necessary (suitable) for an intended assay. Examples of such components include, but are not limited to, oligonucleotides, polymerases (e.g. Taq polymerase), buffers (e.g. Tris buffer), dNTPs, control reagents (e.g. tissue samples, target oligonucleotides for positive and negative controls, etc.), labeling and/or detection reagents (fluorescent dyes such as VIC, FAM), solid supports, manual, illustrative diagrams and/or product information, inhibitors, and packing environment adjusting agents (e.g. ice, desiccating agents). The kit of the present invention may be a partial kit which comprises only a part of the necessary components. In this case, users may provide the remaining components. The kit of the present invention may comprise two or more separate containers, each containing a part of the components to be used. For example, the kit may comprise a first container containing an enzyme and a second container containing an oligonucleotide. Specific examples of the enzyme include a structure-specific cleaving enzyme contained in an appropriate storage buffer or a container. Specific examples of the oligonucleotide include invader oligonucleotides, probe oligonucleotides, target oligonucleotides for use as control, and the like. Alternatively, ore or more reaction components may be provided in such a manner that they are pre-divided into portions of a specific amount. Since such a kit contains components which have already been quantitatively determined for use in one step of the method of the present invention, it is not necessary to re-measure or re-divide. Selected reaction components may also be mixed and divided into portions of a specific amount. It is preferred that reaction components should be pre-divided into portions and contained in a reactor. Specific examples of the reactor include, but are not limited to, reaction tubes or wells, or microtiter plates. It is especially preferable that the pre-divided reaction component should be kept dry in a reactor by means of, for example, dehydration or freeze drying.

### 6. Detection

Using the oligonucleotides prepared as described above as primers, a gene encoding a receptor (template DNA) is amplified with a DNA polymerase. Alternatively, the probe prepared as described above is hybridized to template DNAs to thereby detect those DNAs having the genetic polymorphism of interest. The template DNA may be prepared according to conventional methods, e.g. cesium chloride gradient centrifugation, the SDS lysis method, or phenol/chloroform extraction.

### (1) Detection by PCR

Amplification may be performed by polymerase chain reaction (PCR). Specific examples of useful DNA polymerase include LA Taq DNA polymerase (Takara), Ex Taq polymerase (Takara), Gold Taq polymerase (Perkin Elmer), AmpliTaq (Perkin Elmer), Pfu DNA polymerase (Stratagene) and the like.

Amplification conditions are as follows. Denaturation step at 85-105°C for 10-40 seconds, preferably at 94°C for 20-30 seconds; annealing step at 50-72°C for 20 seconds to 1 minute, preferably at 60°C for 20 seconds to 1 minutes; and extension step at 65-75°C for 1-4 minutes, preferably at 72°C for 2-3 minutes constitute one cycle, and 30 to 40 cycles are performed. However, in order to denature the template DNA and the primers sufficiently, a denaturation step of at 95°C for 1-5 minutes [if Gold Taq polymerase (Perkin Elmer) is used, at least 8-15 minutes, preferably 10-12 minutes] may be added before the start of the above-described amplification cycles. Also, in order to extend the amplified DNA completely, an extension step of at 72°C for 1-10 minutes may be added after the above amplification cycles. Moreover, if the detection of the amplified product is not performed immediately, it is desirable to add a step of storing the amplified product at 4°C to avoid unspecific amplification. Thus, a gene encoding a receptor can be amplified.

Subsequently, the amplified product is subjected to agarose gel electrophoresis, followed by staining with ethidium bromide, SYBR Green solution or the like to thereby detect the amplified product as a band or two to three bands (DNA fragments). Thus, a part of a gene encoding a receptor, containing a genetic polymorphism can be detected as a DNA fragment. Instead of agarose gel electrophoresis, polyacrylamide gel electrophoresis or capillary electrophoresis may be performed. It is also possible to perform PCR using primers labeled in advance with a substance such as fluorescent dye and to detect the amplified product. A detection method which does not require electrophoresis may also be employed; in such a method, the amplified product is bound to a solid support such as a microplate, and a DNA fragment of interest is detected by means of fluorescence, enzyme reaction, or the like.

### (2) Detection by TaqMan PCR

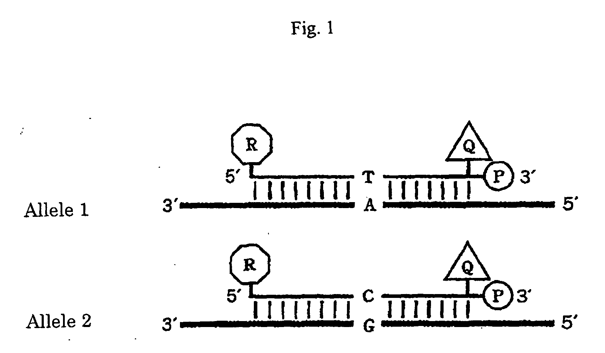

TaqMan PCR is a method using PCR reaction with fluorescently labeled allele-specific oligos and Taq DNA polymerase. The allele-specific oligo used in TaqMan PCR (called "TaqMan probe") may be designed based on the SNP information described above. The 5' end of TaqMan probe is labeled with fluorescence reporter dye R (e.g. FAM or VIC), and at the same time, the 3' end thereof is labeled with quencher Q (quenching substance) (Fig. 1). Thus, under these conditions, fluorescence is not detectable since the quencher absorbs fluorescence energy. Since the 3' end of TaqMan probe is phosphorylated, no extension reaction occurs from TaqMan probe during PCR reaction (Fig. 1). However, when PCR reaction is performed using this TaqMan probe together with Taq DNA polymerase and primers designed so that an SNP-containing region is amplified, the reaction described below occurs.

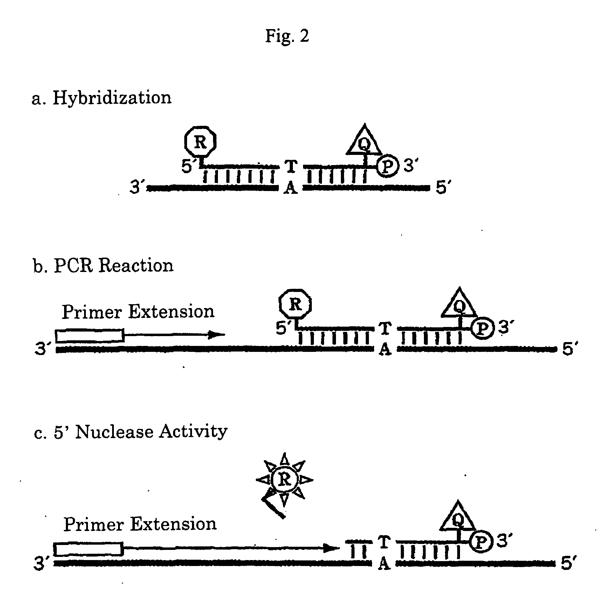

First, a TaqMan probe hybridizes to a specific sequence in the template DNA (Fig. 2a), and at the same time, an extension reaction occurs from a PCR primer (Fig. 2b). At this time, Taq DNA polymerase having 5' nuclease activity cleaves the hybridized TaqMan probe as the extension reaction of PCR primer proceeds. When the TaqMan probe has been cleaved, the fluorescent dye becomes free from the influence of the quencher. Then, fluorescence can be detected (Fig. 2c).

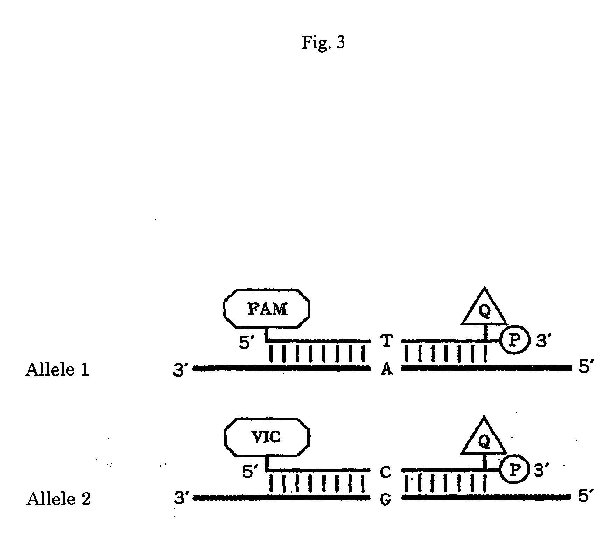

For example, as shown in Fig. 3, two alleles are supposed: one allele has A at the SNP site (allele 1) and the other allele has G at the SNP site (allele 2). A TaqMan probe specific to allele 1 is labeled with FAM and another TaqMan probe specific to allele 2 is labeled with VIC (Fig. 3). These two allele specific oligos are added to PCR reagents, and then TaqMan PCR is performed with a template DNA whose SNP is to be detected. Subsequently, fluorescence intensities of FAM and VIC are determined with a fluorescence detector. When the SNP site of the allele is complementary to the site within TaqMan probe corresponding to the SNP, the probe hybridizes to the allele; and Taq polymerase cleaves the fluorescent dye of the probe, which becomes free form the influence of the quencher. As a result, fluorescence intensity is detected.

If the template is a homozygote of allele 1, strong fluorescence intensity of FAM is recognized but the fluorescence of VIC is hardly recognized. If the template is a heterozygote of allele 1 and allele 2, fluorescence of both FAM and VIC can be detected.

### (3) SNP Detection by the Invader Method

The invader method is a method for detecting SNPs by hybridizing allele-specific oligos to the template. In the invader method, two unlabeled oligos and one fluorescently labeled oligo are used. One of the two unlabeled oligos is called an "allele probe". The allele probe is composed of a region which hybridizes to the genomic DNA (template DNA) to form a complementary double strand, and a region (called "flap") which has a sequence entirely unrelated to the sequence of the template DNA and thus does not hybridize to the genomic DNA. A nucleotide located at the 5' or 3' utmost end of the hybridizable region corresponds to the SNP (panel (a) in Fig. 4A). The above-described flap sequence is an oligonucleotide having a sequence complementary to a FRET probe described later. The other oligo is called an "invader probe". This oligo is designed so that it hybridizes complementarily from the SNP site toward the 3' end of the genomic DNA (panel (b) in Fig. 4A). However, the nucleotide corresponding to the SNP ("N" in panel (b) in Fig. 4A) may be any nucleotide. Thus, when the genomic DNA (the template) is hybridized to the above-described two probes, one nucleotide (N) of the invader probe invades into the SNP site (panel (c) in Fig. 4A). As a result, a triple strand is formed at the SNP site.

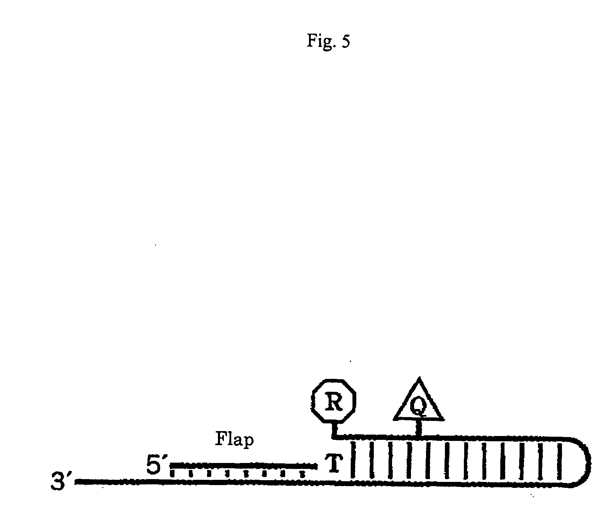

On the other hand, the fluorescently labeled oligo has a sequence completely unrelated to the allele. This sequence is common regardless of the types of SNPs. This probe is called a "FRET" probe (fluorescence resonance energy transfer probe) (Fig. 5). The nucleotide at the 5' end of FRET probe (reporter) is labeled with fluorescent dye R, while quencher Q is linked upstream of the reporter. Therefore, under these conditions, the quencher absorbs the fluorescent dye and no fluorescence is detectable. A certain region of the FRET probe starting from the 5' end reporter nucleotide (designated "region 1") is also designed so that it is complementary to a certain region of the probe located 3' to region 1 (designated "region 2") when region 1 and region 2 are faced with each other. Therefore, region 1 and region 2 form a complementary strand within the FRET probe (Fig. 5). Also, the region located toward 3' end of this complementary strand forming region is designed so that it hybridizes to the flap of the allele probe to thereby form a complementary strand (Fig. 5).

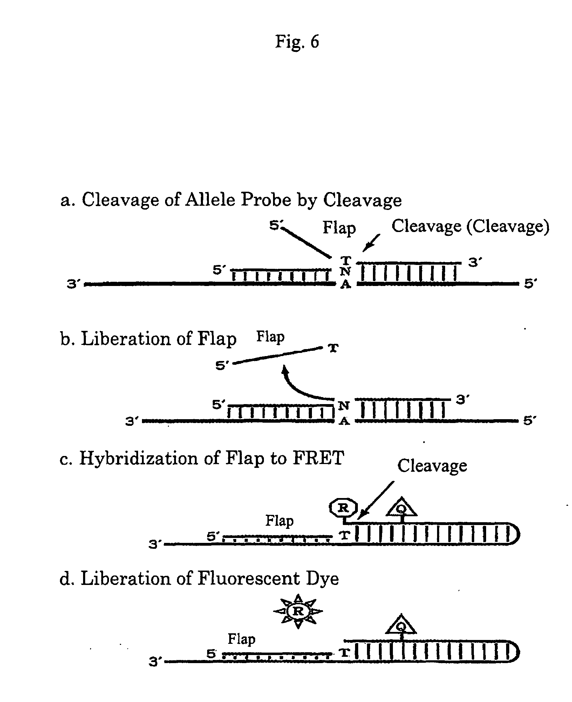

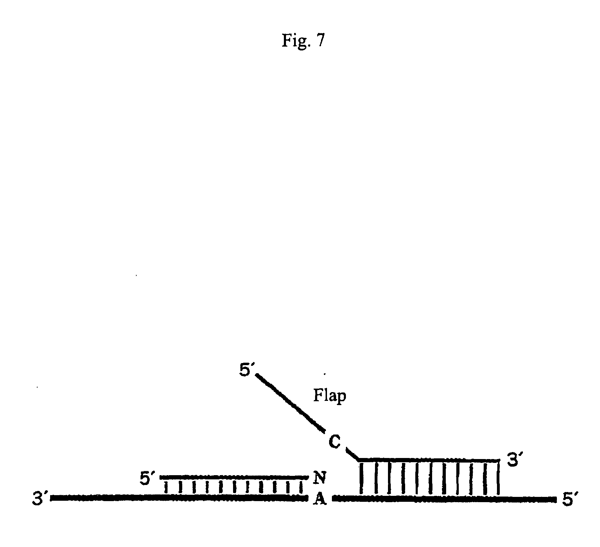

In the invader method, an enzyme called cleavase is used which is one of enzymes (5' nucleotidases) having a unique endonuclease activity of cleaving upon recognition of a special structure of DNA. Cleavase is an enzyme which cleaves the allele probe at a point immediately 3' to the SNP site when the genomic DNA, the allele probe and the invader probe form a triple strand at the SNP site. Therefore, when three nucleotides form a triple strand as shown in panel (c) in Fig. 4A, cleavase recognizes the 5' flap and cuts off this flap. As a result, the structure of this SNP site is recognized by cleavage (panel (a) in Fig. 6), and the allele probe is cut at the site of its flap to liberate the flap (panel (b) in Fig. 6). Subsequently, the flap liberated from the allele probe complementarily binds to the FRET probe since it has a sequence complementary to the FRET probe (panel (c) in Fig. 6). At this time, the SNP site of the flap invades into the portion of the FRET probe which has already formed a complementarily bound region. Cleavase again recognizes this structure and cuts off the nucleotide labeled with the fluorescent dye. The thus cleaved fluorescent dye becomes free from the influence of the quencher and emits fluorescence (panel (d) in Fig. 6). When the SNP does not match the nucleotide corresponding to the SNP in the allele probe, a specific DNA structure recognizable by cleavase is not formed as seen in Fig. 7. Thus, the probe is not cleaved and no fluorescence is detected.

For example, when an SNP is T/C, an invader probe and an allele probe for T, and a FRET probe with a FAM-linked reporter corresponding to the SNP are prepared. Separately, an invader probe and an allele probe for C, and a FRET probe with a VIC-linked reporter corresponding to the SNP are also prepared. Then, all of them are mixed to carry out SNP detection. As a result, if the SNP is T/T homozygous, the fluorescence of FAM is emitted; if the SNP is C/C homozygous, the fluorescence of VIC is emitted; and if the SNP is T/C heterozygous, the fluorescence of both FAM and VIC is emitted. Since FAM and VIC have different fluorescent wavelengths, they can be discriminated. Products labeled with fluorescent dyes can be detected with a fluorescent plate reader or an apparatus for collecting fluorescence data generated during reaction (real-time fluorescence detector). Specific examples of real-time fluorescence detector include ABI 7700 sequence detection system (Applied Biosystems).

### (4) Detection by SniPer Method

In order to detect SNPs by SniPer method, it is possible to discriminate alleles by examining the presence or absence of amplification by RCA. Briefly, the genomic DNA to be used as a template is linearized. Then, a probe is hybridized to this genomic DNA. When the probe sequence and the sequence of the genomic DNA as a template are complementary to each other and form a complementary strand, the genomic DNA can be converted into a circular DNA through ligation reaction. As a result, RCA of the circular DNA proceeds. On the other hand, when the ends of the probe do not match with the genomic DNA, the DNA is not ligated to become a circular DNA. Thus, RCA reaction does not proceed. Therefore, in Sniper method, a single-stranded probe which anneals with the genomic DNA and is circularizable is designed. This single-stranded probe is called a padlock probe. The sequences of the two ends of this padlock probe are designed so that they correspond to the SNP to be detected. Then, this padlock probe and the genomic DNA are mixed for ligation. If the two ends of the padlock probe and the SNP site of the genomic DNA are complementary to each other, the two ends of the padlock probe are joined by ligation, yielding a circular probe. If the two ends of the padlock probe and the SNP site of the genomic DNA are not complementary to each other, the probe does not become circular. Therefore, only those padlock probes which are complementary to the SNP to be detected become circular and are amplified by DNA polymerase. By detecting the presence or absence of this amplification, SNP may be detected. For the detection, synthetic oligonucleotides which have a fluorescent dye and a quencher at their respective ends and also have a hairpin structure are used.

### (5) Detection by MALDI-TOF/MS Method

MALDI-TOF/MS (Matrix Assisted Laser Disorption-Time of Flight/Mass Spectrometry) is a method using a mass spectrometer in SNP typing. This method is composed of the following steps.

### (i) PCR Amplification and Purification of SNP-Containing DNA Fragments

PCR primers are designed so that there is no overlapping between them and the nucleotides of SNP site. Then, DNA fragments are amplified. The amplified fragments are purified from the amplification reaction product by treatment with exonuclease, alkaline phosphatase, etc. to remove primers, dNTPs, etc.

### (ii) Primer Extension (Thermal Cycling) and Purification

Ten-fold or more primers are added to the template of the target region (which is the PCR product), and primer extension is performed by thermal cycling. The primers used here are designed so that their 3' ends are adjacent to the nucleotide of the SNP site. The length of the primer is 15 to 30 nucleotides, preferably 20 to 25 nucleotides. When multiplex reaction is performed, a sequence not complementary to the template is added to the 5' end. Thermal cycling is performed between the two temperatures of at 85-105°C (preferably 94°C) and at 35-40°C (preferably 37°C) for 20 to 30 cycles (preferably 25 cycles). The resultant reaction products are purified with a purification kit or the like to make them fit for mass spectrometer.

### (iii) Mass Spectrometry of DNA with Mass Spectrometer

The purified extension reaction product is applied to a mass spectrometer to determine the mass of the objective product. Briefly, the purified product is mixed with a matrix, and 0.5-1.0 l1 of the mixture is spotted on MALDI plate. After drying the plate, laser light is applied to the sample to prepare spectrograms.

### (6) Detection by DNA Sequencing Method

In the present invention, polymorphisms may be detected by using single nucleotide extension reactions. Briefly, four types of dideoxynucleotides labeled with different fluorescent compounds are added to a reaction system containing a gene of interest. Then, single nucleotide extension reactions are performed. In this case, the nucleotide to be extended is the polymorphic site. Also, two reactions of DNA synthesis termination and the fluorescent labeling of the 3' end of DNA molecules are operated. Four types of reaction solutions are subjected to electrophoresis on the same lane of a sequencing gel or on capillary. Difference in the fluorescent dyes used for labeling is detected with a fluorescence detector to thereby sequence the DNA band. Alternatively, the one-nucleotide extended oligonucleotide is examined with a fluorescence detection system or a mass spectrometry system or the like to thereby determine which nucleotide was extended using the difference in the fluorescent dyes. Instead of fluorescently labeled dideoxynucleotides, primers may be fluorescently labeled and used with unlabeled dideoxynucleotides.

### (7) Detection in DNA Microarray

DNA microarrays are solid supports onto which nucleotide probes are immobilized, and they include DNA chips, gene chips, microchips, beads arrays, and the like.

As a specific example of DNA microarray (e.g. DNA chip) assay, GeneChip assay (Affymetrix; US Patent Nos. 6,045,996; 5,925,525; and 5,858,659) may be given. GeneChip technology uses small sized, high density microarrays of oligonucleotide probes affixed to chips. Probe arrays are manufactured, for example, by the light irradiation chemical synthesis method (Affymetrix) which is a combination of solid chemical synthesis method and photolithography production technology used in the semiconductor industry. High density arrays to which oligonucleotide probes are affixed on designed place can be constructed by using photolithography masks in order to make the boundary of the chemical reaction site of chips definite and by performing a specific chemical synthesis step. Multiple-probe arrays are synthesized simultaneously on a large glass baseboard. Subsequently, this baseboard is dried, and individual probe arrays are packed in injection-molded plastic cartridges. This cartridge protects the array from the outer environment and also serves as a hybridization chamber.

First, a polynucleotide to be analyzed is isolated, amplified by PCR, and labeled with a fluorescent reporter group. Then, the labeled DNA is incubated with an array using a fluid station. This array is inserted into a scanner to detect a hybridization pattern. Hybridization data are collected as luminescence from fluorescent reporter group bound to the probe array (i.e. taken into the target sequence). Generally, probes which completely matched with the target sequence generate stronger signal than those probes which have portions not matching with the target - sequence. Since the sequences and locations of individual probes on the array are known, it is possible to determine the sequence of the target polynucleotide reacted with the probe array on the basis of complementation.

In the present invention, it is also possible to use DNA microchips with electrically captured probes (Nanogen; see, for example, US Patents Nos. 6,017,696; 6,068,818; and 6,051,380). By using microelectronics, the technology of Nanogen is capable of transferring charged molecules to and from specific test sites on semiconductor microchips and concentrating them. DNA capturing type probes specific to certain SNPs or variations are arranged on specific sites on microchips electrically or assigned addresses. Since DNA is strongly negatively charged, it is capable of moving electronically to a positively charged area.

First, the test site or a row of the test site on a microchip is electronically activated with positive charge. Then, a solution containing DNA probes is introduced onto the microchip. Since negatively charged probes quickly moves to a positively charged site, probes are concentrated at this site on the microchip and chemically bind thereto. This microchip is washed, and another DNA probe solution is added to the microchip to allow specific binding of DNA probes to the chip.

Subsequently, whether the target DNA molecule is present in a test sample or not is analyzed. This analysis is performed by judging the type of the DNA capturing probe which has hybridized to a complementary DNA in a test sample. As a test sample, for example, a gene of interest which has been amplified by PCR may be given. By using electric charge, target molecules may be moved to one or more test sites on the microchip and concentrated. As a result of electronic concentration of the sample DNA at each test site, the hybridization between the sample DNA and a capturing probe complementary thereto is performed quickly. For example, as a result of these operations, hybridization occurs in several minutes. In order to remove unbound DNA or non-specifically bound DNA from each test site, the polarity or electric charge of the site is converted to negative charge to thereby return the unbound DNA or non-specifically bound DNA into the solution. In this method, specific binding can be detected, for example, with a fluorescence scanner utilizing laser.

Further, in the present invention, it is also possible to use an array technology utilizing fluid separation phenomenon on a plane surface (chip) because of difference in surface tensions (ProtoGene; see, for example. US Patents Nos. 6,001,311; 5,985,551; and 5,474,796). The technology of ProtoGene is based on a fact that fluids are separated from each other on a plane surface because of difference in surface tensions given by chemical coating. Since oligonucleotide probes may be separated based on the above-mentioned principle, it is possible to synthesize probes directly on a chip by the ink-jet printing of a reagent containing probes. An array having reaction sites defined by surface tension is mounted on X/Y movable stage located under one set (4) of piezoelectric nozzles. Each piezoelectric nozzle contains four standard DNA nucleotides, respectively. This movable stage moves along each row of the array to supply an appropriate reagent (e.g. amidite) to each reaction site. The entire surface of the array is soaked in a reagent common to the test sites in the array and then in a washing solution. Subsequently, the array is rotated to remove these solutions.

DNA probes specific to the SNPs or variations to be detected are affixed to a chip using the technology of Protogene. Then, the chip is contacted with PCR-amplified gene of interest. After hybridization, unbound DNA is removed, followed by detection of hybridization using an appropriate method.

Further, it is possible to detect polymorphisms using "bead arrays" (Illumina Inc.; see, for example, PCT International Publication Nos. WO 99/67641 and WO 00/39587). Illumina Inc. utilizes Bead Array technology which uses a combination of optical fiber bundles and beads that undergo self-association with the array. Each optical fiber bundle has several millions of fibers depending on the diameter of the bundle. Beads are coated with oligonucleotides specific for the detection of certain SNPs or variations. Various types of beads are mixed in specific amounts to allow the formation of an array-specific pool. For assay, a bead array is contacted with a sample prepared from a subject. Then, hybridization is detected by any appropriate method.

### 7. Evaluation of Drugs

In the present invention, it is possible to evaluate the efficacy and safety of a drug intermediated by the receptor, from the results of detection of SNP and like that obtained as described above.

Evaluation of drugs may be performed by typing system. Briefly, according to any one of the detection methods described above, allele frequencies between toxicity (side effect) occurrence group and non-occurrence group are compared. A polymorphism which brings about difference in allele frequencies between the two groups is selected as a marker for recognizing the occurrence of toxicity. As a statistical test, usually chi square test is carried out, but other statistical processing such as Fisher test may also be used. It is also possible to allow that binding activity of the ligand to the receptor, the strength of the inhibitory effect by the drug to the binding activity, cell response under stimulating the cell and the like having the receptor by the ligand and the inhibitory activity by the drug to the effect, the expression level of the receptor or the like reflect to the binding level of the ligand, the binding level of the anti-receptor antibody etc using these results as indices. With respect to all genetic polymorphisms, the relation of cause and effect with the action or toxicity is examined. Then, only those genetic polymorphism sites that show correlation with the action or toxicity are selected. Allele pattern can be examined by preparing in advance all probes or primers for analyzing the genetic polymorphisms and reagents necessary for each technique in reaction plates, cards, glass baseboards or the like, and adding thereto the genomic DNA of a human subject for reaction. When the subject has a genetic polymorphism which has correlation with the action or toxicity, it is possible to predict whether the drug exhibits effect or toxicity in that subject. The efficacy of a drug may be evaluated in a similar manner. Also, genetic polymorphisms which correlate with side effect or efficacy vary depending on drugs. Therefore, by conducting typing using correlating genetic polymorphisms for each drug, it becomes possible to predict the efficacy or side effect of the relevant drug.

Using this, the frequency of the relevant genetic polymorphism is compared with efficacy/non-efficacy or presence/absence of side effect. When there is difference in allele frequency, a judgment on the relevant drug can be made.

For example, if the results of analysis of an SNP in persons who showed toxicity (side effect) upon administration of drug A have revealed statistically that 90% of those persons have T/T (e.g. fluorescence intensity of FAM was detected), and if the results of analysis of the SNP in persons who did not show toxicity (side effect) have revealed that only 10% of those persons have T/T and 90% of them have C/C, drug A can be evaluated that it should not be administered to persons with T/T.

### 8. Screening for Drugs.

The genetic polymorphism information obtained as described above in the present invention may be compared with genetic polymorphism information in a gene of a subject encoding a corresponding receptor or a complementary sequence thereto. By doing so, the above information may be used as an indicator for analyzing the efficacy and safety of a drug acting on the receptor (i.e. a drug intermediated by the receptor). It is also possible to analyze individual difference on the efficacy and safety of a drug using the above-described genetic polymorphism information. Therefore, the genetic polymorphism information obtained in the present invention serves as an information source for selecting most effective drug to treat a disease or for selecting the drug to be used and/or the dose of the drug.

As a method, the evaluation method described in "7. Evaluation of Drugs" may be used. Briefly, it can be said that the genetic polymorphisms which were recognized to be correlating with side effect or efficacy in the preceding sub-section give influence upon the ability of a ligand to bind to the relevant receptor, the signal transduction ability of the receptor, and the level of the receptor expression derived from transcription/translation. Also the genetic polymorphisms have any relation of cause and effect with the mechanism of occurrence of side effect or efficacy even indirectly. The sensitivity of a drug is examined and confirmed by a pharmaceutical manufacturer or the like in pre-clinical test or clinical test. Therefore, if a polymorphism which correlates with severe side effect exists among the genetic polymorphisms located in the gene encoding the enzyme, it is possible to delete it or to use the drug conditionally. With respect to efficacy, a similar evaluation can be made. From such information on side effect and efficacy, drug screening becomes possible.

Further, by conducting genetic polymorphism frequency analysis on cases of volunteers with side effect occurrence and cases without side effect occurrence in clinical tests (from phase I to phase III tests), it becomes possible to detect new genetic polymorphisms other than the above-mentioned polymorphism which correlate with side effect or efficacy. By examining such polymorphisms in the same manner as described above, drug screening becomes possible.

### BRIEF DESCRIPTION OF THE DRAWINGS

Fig. 1 is a diagram showing TaqMan probes.
Fig. 2 is a diagram showing an outline of TaqMan PCR method.
Fig. 3 is a diagram showing probes labeled with a fluorescent dye.
Fig. 4A is a diagram showing an outline of the invader method.
Fig. 4B is a diagram showing locational relationships between an invader probe and an allele probe.
Fig. 4C is a diagram showing locational relationships between an invader probe and an allele probe.
Fig. 5 is a diagram showing a FRET probe.
Fig. 6 is a diagram showing an outline of the invader method.
Fig. 7 is a diagram showing a probe that does not match with an allele.

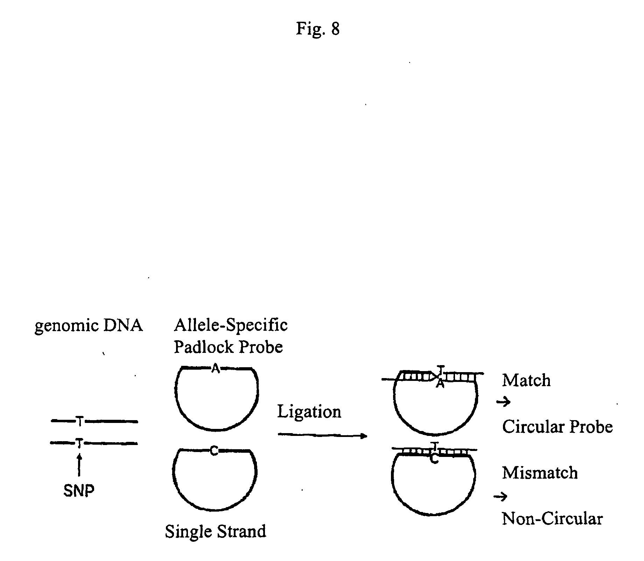

Fig. 8 is a diagram showing an outline of allele discrimination by ligation reaction.
Fig. 9 is a diagram showing the structure of *CD20* (CD20 antigen gene) and the locations of SNPs.
   Accession No.: AP001034.4
Fig. 10 is a diagram showing the structure of *CD33* [CD33 antigen (gp67) gene] and the locations of SNPs.
   Accession No.: AC063977.5
Fig. 11 is a diagram showing the structure of *CSF3R* [colony stimulating factor 3 receptor (granulocyte) gene] and the locations of SNPs.
   □@ Accession No.: AL445245.3
Fig. 12 is a diagram showing the structure of *IL1R1* (interleukin 1 receptor, type I, gene) and the locations of SNPs.
   Accession No.: AC007271.3
Fig. 13 is a diagram showing the structure of *IL1R2* (interleukin 1 receptor, type II, gene) and the locations of SNPs.
   Accession Nos.: AC005035.1, AC007165.3
Fig. 14 is a diagram showing the structure of *IL2R* (interleukin-2 receptor gene) and the locations of SNPs.
   Accession Nos.: AL157395.16, AL137186.18
Fig. 15 is a diagram showing the structure of *HER2* (c-erb-B-2 gene) and the locations of SNPs.
   Accession No.: AC079199.3
Fig. 16 is a diagram showing the structure of *IFNAR1* [interferon (alpha, beta and omega) receptor 1 gene] and the locations of SNPs.
   Accession No.: AP001716.1
Fig. 17 is a diagram showing the structure of *PGR* (progesterone receptor gene) and the locations of SNPs.
   Accession No.: AC020735.5
Fig. 18 is a diagram showing the structure of *ACTH* [melanocortin 2 receptor (MC2R) gene] and the locations of SNPs.
   Accession Nos.: AP001086.4 and Y10259.1
Fig. 19 is a diagram showing the structure of *ICAM1* [intercellular adhesion molecule 1 (CD54), human rhinovirus receptor gene] and the locations of SNPs.
   Accession No.: AC011511.9
Fig. 20 is a diagram showing the structure of *VCAM1* (vascular cell adhesion molecule 1 gene) and the locations of SNPs.
   Accession No.: AL157715.5
Fig. 21 is a diagram showing the structure of *ITGB2* [leukocyte integrin, beta 2 (antigen CD18 (p95); lymphocyte function-associated antigen 1; macrophage antigen 1 (mac-1) beta subunit) gene] and locations of SNPs.
   Accession No.: AL163300.2
Fig. 22 is a diagram showing the structure of *PTGDR* [prostaglandin D2 receptor (DP) gene] and the locations of SNPs.
   Accession No.: AL355833.4
Fig. 23 is a diagram showing the structure of *PTGER1* [prostaglandin E receptor 1 (subtype EP1), 42kD gene] and the locations of SNPs.
   Accession No.: AC008569.6
Fig. 24 is a diagram showing the structure of *PTGER2* [prostaglandin E receptor 2 (subtype EP2), 53kD gene] and the locations of SNPs.
   Accession No.: AL365475.1
Fig. 25 is a diagram showing the structure of *PTGER3* (prostaglandin E receptor 3 gene) and the locations of SNPs.
   Accession Nos.: AL031429.11, AL158087.11
Fig. 26 is a diagram showing the structure of *PTGFR* [prostaglandin F receptor (FP) gene] and the locations of SNPs.
   Accession No.: AL136324.6
Fig. 27 is a diagram showing the structure of *GNA12* (thromboxane A2 receptor/G protein alpha 12 gene) and the locations of SNPs.
   Accession No.: AC006028.3
Fig. 28 is a diagram showing the structure of *TBXA2R* (thromboxane A2 receptor gene) and the locations of SNPs.
   Accession No.: AC005175.1
Fig. 29 is a diagram showing the structure of *BLTR2* (seven transmembrane receptor BLTR2 gene; leukotriene B4 receptor BLT2 gene) and the locations of SNPs.
   Accession No.: AL096870.5
Fig. 30 is a diagram showing the structure of *CYSLT1* (cysteinyl leukotriene receptor 1 gene) and the locations of SNPs.
   Accession No.: AL445202.21
Fig. 31 is a diagram showing the structure of *CYSLT2* (cysteinyl leukotriene receptor 2 gene) and the locations of SNPs.
   Accession No.: AL137118.20
Fig. 32 is a diagram showing the structure of *PTAFR* (platelet-activating factor receptor gene) and the location of SNP.
   Accession No.: AC027421.3
Fig. 33 is a diagram showing the structure of *BDKRB1* (bradykinin receptor B1 gene) and the locations of SNPs.
   Accession No.: AL355102.5
Fig. 34 is a diagram showing the structure of *BDKRB2* (bradykinin receptor B2 gene) and the locations of SNPs.
   Accession No.: AF378542.2
Fig. 35 is a diagram showing the structure of *ADRB1* (adrenergic, beta-1-, receptor gene) and the locations of SNPs.
   Accession No.: AC005886.2
Fig. 36 is a diagram showing the structure of *ADRB2* (adrenergic, beta-2-, receptor, surface gene) and the locations of SNPs.
   Accession No.: AC011334.4
Fig. 37 is a diagram showing the structure of *HRH1* (histamine H1 receptor gene) and the locations of SNPs.
   Accession No.: AC020750.3
Fig. 38 is a diagram showing the structure of *HRH2* (histamine H2 receptor gene) and the locations of SNPs.
   Accession No.: AB023486.1
Fig. 39 is a diagram showing the structure of □@*HRH3* (histamine H3 receptor gene) and the locations of SNPs.
   Accession No.: AL078633.32
Fig. 40 is a diagram showing the structure of *HTR3A* [5-hydroxytryptamine (serotonin) receptor 3A gene] and the locations of SNPs.
   Accession No.: AP001874.3
Fig. 41 is a diagram showing the structure of *AGTR1* (angiotensin receptor 1 gene) and the locations of SNPs.
   Accession No.: AC024897.23
Fig. 42 is a diagram showing the structure of *AGTRL1* (angiotensin receptor-like 1 gene) and the locations of SNPs.
   Accession No.: AP001786.4
Fig. 43 is a diagram showing the structure of *AGTR2* (angiotensin receptor 2 gene) and the locations of SNPs.
   Accession No.: AC069480.2
Fig. 44 is a diagram showing the structure of *AVPR1A* (arginine vasopressin receptor 1A gene) and the locations of SNPs.
   Accession No.: AC025525.3
Fig. 45 is a diagram showing the structure of *AVPR2* (arginine vasopressin receptor 2 gene) and the locations of SNPs.
   Accession No.: U52112.1
Fig. 46 is a diagram showing the structure of *PTGIR* [prostaglandin 12 (prostacyclin) receptor (IP) gene] and the locations of SNPs.
   Accession No.: AC025983.3
Fig. 47 is a diagram showing the structure of *DRD1* (dopamine receptor D1 gene) and the locations of SNPs.
   Accession No.: AC091393.1
Fig. 48 is a diagram showing the structure of *ITGA2B* [integrin, alpha 2b (platelet glycoprotein IIb of IIb/IIIa complex, antigen CD41B) gene] and the locations of SNPs.
   Accession No.: AC019152.5
Fig. 49 is a diagram showing the structure of *FOLR1* (folate receptor 1 gene) and the locations of SNPs.
   Accession No.: U20391.1
Fig. 50 is a diagram showing the structure of *TNFR1* (tumor necrosis factor receptor 1 gene) and the locations of SNPs.
   Accession No.: AC006057.5
Fig. 51 is a diagram showing the structure of *ADORA2A* (adenosine A2 receptor gene) and the locations of SNPs.
   Accession No.: AP000355.1
Fig. 52 is a diagram showing the structure of *AVPR1B* (arginine vasopressin receptor 1B gene) and the locations of SNPs.
   Accession No.: AF152238.1
Fig. 53 is a diagram showing the structure of *MC2R* (melanocortin 2 receptor gene) and the locations of SNPs.
   Accession Nos.: AP001086.4 and Y10259.1
Fig. 54 is a diagram showing the structure of *ADORA1* (adenosine A1 receptor gene) and the locations of SNPs.
   Accession No.: AC105940.2
Fig. 55 is a diagram showing the structure of *ADORA2B* (adenosine A2b receptor gene) and the locations of SNPs.
   Accession No.: AC006251.3
Fig. 56 is a diagram showing the structure of *ADORA3* (adenosine A3 receptor gene) and the locations of SNPs.
   Accession No.: AL390195.10
Fig. 57 is a diagram showing the structure of *ADRA1A* (adrenergic, alpha-1A-, receptor gene) and the locations of SNPs.
   Accession No.: AC025712.4
Fig. 58 is a diagram showing the structure of *ADRA2A* (adrenergic, alpha-2A-, receptor gene) and the locations of SNPs.
   Accession No.□FAL158163.11
Fig. 59 is a diagram showing the structure of *ADRA2B* (adrenergic, alpha-2B-, receptor gene) and the locations of SNPs.
   Accession No.: AC092603.2
Fig. 60 is a diagram showing the structure of *EDG1* (endothelial differentiation, sphingolipid G-protein-coupled receptor, 1 gene) and the locations of SNPs.
   Accession No.: AL109741.19
Fig. 61 is a diagram showing the structure of *EDG4* (endothelial differentiation, lysophosphatidic acid G-protein-coupled receptor, 4 gene) and the locations of SNPs.
   Accession No.: AC011458.7
Fig. 62 is a diagram showing the structure of *EDG5* (endothelial differentiation, sphingolipid G-protein-coupled receptor, 5 gene) and the locations of SNPs.
   Accession No.: AC011511.12
Fig. 63 is a diagram showing the structure of *GPR1* (G protein-coupled receptor 1 gene) and the location of SNP.
   Accession No.: AC007383.4
Fig. 64 is a diagram showing the structure of *GPR2* (G protein-coupled receptor 2 gene) and the location of SNP.
   Accession No.: AC027146.1
Fig. 65 is a diagram showing the structure of *GPR3* (G protein-coupled receptor 3 gene) and the locations of SNPs.
   Accession No.: AL096774.9
Fig. 66 is a diagram showing the structure of *GPR4* (G protein-coupled receptor 4 gene) and the locations of SNPs.
   Accession No.: AC011480.3
Fig. 67 is a diagram showing the structure of *MC1R* (melanocortin 1 receptor gene) and the locations of SNPs.
   Accession No.: AC092143.3
Fig. 68 is a diagram showing the structure of *MC3R* (melanocortin 3 receptor gene) and the locations of SNPs.
   Accession No.: AL139824.22
Fig. 69 is a diagram showing the structure of *MC4R* (melanocortin 4 receptor gene) and the locations of SNPs.
   Accession No.: AC091576.11
Fig. 70 is a diagram showing the structure of *OXTR* (oxytocin receptor gene) and the locations of SNPs.
   Accession No.: AF176315.2
Fig. 71 is a diagram showing the structure of *SSTR1* (somatostatin receptor 1 gene) and the locations of SNPs.
   Accession No.: AL450109.3
Fig. 72 is a diagram showing the structure of *SSTR3* (somatostatin receptor 3 gene) and the locations of SNPs.
   Accession No.: Z82188.2
Fig. 73 is a diagram showing the structure of *GPR10* (G protein-coupled receptor 10 gene) and the locations of SNPs.
   Accession No.: AC067895.2

### BEST MODE FOR CARRYING OUT THE INVENTION

Hereinbelow, the present invention will be described more specifically with reference to the following Example. However, the technical scope of the present invention is not limited to the Example.

### [EXAMPLE 1] Acquisition of SNP Information

### (1) DNA Extraction

Blood samples were collected in the presence of EDTA from 48 individuals who have no kinship relation with one another. DNA extraction was carried as described below according to the method described in "Genome Analysis Laboratory Manual" (Yusuke Nakamura (ed.), Springer Verlag Tokyo).

Blood sample (10 ml) was transferred to a 50 ml Falcon tube and centrifuged at room temperature at 3000 rpm for 5 minutes. After removal of the supernatant (serum) with a pipette, 30 ml of RBC lysis buffer (10mM NH₄HCO₃, 144mM NH₃Cl) was added and mixed until the precipitate became loosened. Then, the mixture was left at room temperature for 20 minutes. After centrifugation at room temperature at 3000 rpm for 5 minutes, the supernatant (serum) was discarded with a pipette to obtain a pellet of white blood cells. RBC lysis buffer (30 ml) was added thereto, and the above-described operations were repeated twice. To the resultant white blood cell pellet, 4 ml of Proteinase K buffer (50mM Tris-HCl (pH7.4), 100mM NaCl, 1mM EDTA (pH8.0)), 200 l1 of 10% SDS, and 200 l1 of 10 mg/ml Proteinase K were added and mixed by inversion. The resultant mixture was left overnight stationery at 37°C. Subsequently, 4 ml of phenol was added to the mixture, which was then mixed slowly by inversion for 4 hours in a rotator (Rotator T-50, Taitec). After centrifugation at room temperature at 3000 rpm for 10 minutes, the resultant upper layer was collected into a fresh tube. Four milliliters of phenol/chloroform/isoamyl alcohol (25:24:1 in volume ratio) was added to the tube and mixed by inversion for 2 hours in the same manner as described above. Then, the mixture was centrifuged. The resultant upper layer was collected into a fresh tube, to which 4 ml of chloroform/isoamyl alcohol (24:1 in volume ratio) was added and mixed by inversion for 30b minutes in the same manner as described above. Then, the mixture was centrifuged. The resultant upper layer was collected into a fresh tube, to which 400 ìl of 8M ammonium acetate and 4 ml of isopropanol were added and mixed by inversion. Thread-like white deposit (DNA) was recovered into a 2 ml tube, to which 70% ethanol (1 ml) was added and mixed by inversion. The DNA was recovered into a fresh 2 ml tube and air-dried. Then, 500 ìl of TE solution (10mM Tris-HCl (pH7.4), 1mM EDTA (pH7.4)) was added for lysis, to thereby obtain a genomic DNA sample.

### (2) PCR

Genomic sequences were obtained from GenBank DNA database. After removal of repeat sequences using RepMask computer program, PCR primers were designed so that PCR products have a length of about 1 kb. As genomic DNA, DNA samples obtained from 48 individuals who have no kinship relation with one another and prepared to have the same concentration were used. DNA samples derived from three individuals each were mixed in a tube in equal amounts. Of this mixture, 60 ng was used in PCR. PCR was performed with Ex-Taq (2.5 U; Takara) using GeneAmp PCR System 9700 (PE Applied Biosystems). Following a reaction at 94°C for 2 minutes, 35 cycles of denaturation at 94°C for 30 seconds, annealing at 60°C or 55°C for 30 seconds and extension at 72°C for 1 minute were performed.

### (3) Sequencing

PCR products were purified with Arraylt (Telechem) and subjected to sequencing reaction using BigDye Terminator RR Mix (PE Applied Biosystems). Briefly, following a reaction at 96°C for 2 minutes, 25 cycles of denaturation at 96°C for 20 seconds, annealing at 50°C for 30 seconds and extension at 60°C for 4 minutes were performed using GeneAmp PCR System 9700 (PE Applied Biosystems). After the sequencing reaction, sequences were analyzed with ABI PRISM 3700 DNA Analyzer.

### (4) Detection of SNPs

PolyPhred computer program (Nickerson et al., 1997, Nucleic Acids Res., 25, 2745-2751) was used for the detection and analysis of SNPs.

### (5) Results

The results as shown in Table 1 were obtained on SNPs. Figs. 9 to 73 show the designations, abbreviations and GenBank database Accession Nos. of the analyzed receptors, the structures of the genes encoding them, and the locations of SNPs. In Figs. 9 to 73, exons are indicated as open boxes or black lines on the relevant gene expressed as a horizontal line. The locations of SNPs are indicated above the gene with solid lines provided with numbers. In Figs. 9 to 40 and 49 to 73, the locations of polymorphisms other than SNP are indicated below the relevant gene with solid lines provided with numbers.

### INDUSTRIAL APPLICABILITY

According to the present invention, methods for analyzing SNPs are provided. According to the methods of the invention, it becomes possible to select appropriate drugs for target diseases. Thus, the methods of the invention are extremely useful.

### SEQUENCE LISTING FREE TEXT

SEQ ID NO: 21: n represents 10 to 12 times repetition of t (location: 21).
SEQ ID NO: 22: n represents g or deletion (location: 21).
SEQ ID NO: 23: n represents a or deletion (location: 21).
SEQ ID NO: 24: n represents 8 to 10 times repetition of ca (location: 21).
SEQ ID NO: 25: n represents an insertion of gact (location: 21).
SEQ ID NO: 27: n represents 8 to 9 times repetition of a (location: 21).
SEQ ID NO: 43: n represents 8 to 10 times of repetition of c (location: 21).
SEQ ID NO: 44: n represents c or deletion (location: 21).
SEQ ID NO: 45: n represents a or deletion (location: 21).
SEQ ID NO: 46: n represents an insertion of a (location: 21).
SEQ ID NO: 85: n represents a or deletion (location: 21).
SEQ ID NO: 86: n represents an insertion of g (location: 21).
SEQ ID NO: 88: n represents 6 to 11 times repetition of aaac (location: 21).
SEQ ID NO: 89: n represents 9 to 12 times repetition of a (location: 21).
SEQ ID NO: 90: n represents 11 to 13 times repetition of t (location: 21).
SEQ ID NO: 91: n represents 10 to 14 times repetition of t (location: 21).
SEQ ID NO: 92: n represents 20 to 26 times repetition of t (location: 21).
SEQ ID NO: 93: n represents t or deletion (location: 21).
SEQ ID NO: 94: n represents 4 to 6 times repetition of ct (location: 21).
SEQ ID NO: 95: n represents 10 to 12 times repetition of a (location: 21).
SEQ ID NO: 96: n represents 7 to 11 times repetition of g (location: 21).
SEQ ID NO: 129: n represents tt or deletion (location: 21).
SEQ ID NO: 130: n represents 10 to 11 times repetition of ga (location: 21).
SEQ ID NO: 131: n represents tt or deletion (location: 21).
SEQ ID NO: 132: n represents 19 to 22 times repetition of t (location: 21).
SEQ ID NO: 133: n represents t or deletion (location: 21).
SEQ ID NO: 134: n represents 15 to 18 times repetition of a (location: 21).
SEQ ID NO: 135: n represents 7 to 8 times repetition of a (location: 21).
SEQ ID NO: 169: n represents g or deletion (location: 21).
SEQ ID NO: 170: n represents g or deletion (location: 21).
SEQ ID NO: 171: n represents 12 to 14 times repetition of a or deletion (location: 21).
SEQ ID NO: 172: n represents an insertion of t (location: 21).
SEQ ID NO: 174: n represents an insertion of t (location: 21).
SEQ ID NO: 176: n represents a or deletion (location: 21).
SEQ ID NO: 177: n represents g or deletion (location: 21).
SEQ ID NO: 178: n represents g or deletion (location: 21).
SEQ ID NO: 190: n represents 5 to 14 times repetition of gt (location: 21).
SEQ ID NO: 191: n represents 9 to 11 times repetition of a (location: 21).
SEQ ID NO: 196: n represents an insertion of tg (location: 21).
SEQ ID NO: 198: n represents t or deletion (location: 21).
SEQ ID NO: 199: n represents 7 to 8 times repetition of ac (location: 21).
SEQ ID NO: 219: n represents c or deletion (location: 21).
SEQ ID NO: 220: n represents an insertion of tt (location: 21).
SEQ ID NO: 222: n represents 3 to 30 times repetition of ga and 3 to 30 times repetition of gt (location: 21).
SEQ ID NO: 231: n represents 12 to 14 times repetition of t (location: 21).
SEQ ID NO: 249: n represents gcag or deletion (location: 21).
SEQ ID NO: 250: n represents ca or deletion (location: 21).
SEQ ID NO: 251: n represents t or deletion (location: 21).
SEQ ID NO: 252: n represents t or deletion (location: 21).
SEQ ID NO: 253: n represents 12 to 15 times repetition of a (location: 21).
SEQ ID NO: 254: n represents 10 to 12 times repetition of t (location: 21).
SEQ ID NO: 255: n represents t or deletion (location: 21).
SEQ ID NO: 256: n represents t or deletion (location: 21).
SEQ ID NO: 295: n represents c or deletion (location: 21).
SEQ ID NO: 296: n represents ag or deletion (location: 21).
SEQ ID NO: 297: n represents an insertion of c (location: 21).
SEQ ID NO: 299: n represents ttcc or deletion (location: 21).
SEQ ID NO: 300: n represents c or deletion (location: 21).
SEQ ID NO: 313: n represents tcc or deletion (location: 21).
SEQ ID NO: 314: n represents 10 to 11 times repetition of a (location: 21).
SEQ ID NO: 315: n represents tcagg or deletion (location: 21).
SEQ ID NO: 316: n represents 11 to 12 times repetition of t (location: 21).
SEQ ID NO: 330: n represents gg or deletion (location: 21).
SEQ ID NO: 331: n represents an insertion of tg (location: 21).
SEQ ID NO: 333: n represents 7 to 8 times repetition of t (location: 21).
SEQ ID NO: 344: n represents 6 to 9 times repetition of c (location: 21).
SEQ ID NO: 345: n represents a or deletion (location: 21).
SEQ ID NO: 346: n represents 9 to 10 times repetition of t (location: 21).
SEQ ID NO: 347: n represents a or deletion (location: 21).
SEQ ID NO: 348: n represents c or deletion (location: 21).
SEQ ID NO: 349: n represents ttta or deletion (location: 21).
SEQ ID NO: 350: n represents 12 to 14 times repetition of a (location: 21).
SEQ ID NO: 478: n represents actt or deletion (location: 21).
SEQ ID NO: 479: n represents an insertion of ca (location: 21).
SEQ ID NO: 481: n represents 7 to 8 times repetition of t (location: 21).
SEQ ID NO: 482: n represents c or deletion (location: 21).
SEQ ID NO: 483: n represents 11 to 13 times repetition of t (location: 21).
SEQ ID NO: 484: n represents 10 to 12 times repetition of t (location: 21).
SEQ ID NO: 485: n represents a or deletion (location: 21).
SEQ ID NO: 486: n represents 10 to 12 times repetition of t (location: 21).
SEQ ID NO: 487: n represents 8 to 9 times repetition of t (location: 21).
SEQ ID NO: 488: n represents aatt or deletion (location: 21).
SEQ ID NO: 489: n represents an insertion of t (location: 21).
SEQ ID NO: 491: n represents 9 to 11 times repetition of at (location: 21).
SEQ ID NO: 492: n represents t or deletion (location: 21).
SEQ ID NO: 493: n represents an insertion of cact (location: 21).
SEQ ID NO: 495: n represents t or deletion (location: 21).
SEQ ID NO: 496: n represents gaa or deletion (location: 21).
SEQ ID NO: 497: n represents an insertion of t (location: 21).
SEQ ID NO: 499: n represents t or deletion (location: 21).
SEQ ID NO: 500: n represents a or deletion (location: 21).
SEQ ID NO: 501: n represents t or deletion (location: 21).
SEQ ID NO: 502: n represents 10 to 15 times repetition of a (location: 21).
SEQ ID NO: 503: n represents a or deletion (location: 21).
SEQ ID NO: 504: n represents an insertion of a (location: 21).
SEQ ID NO: 506: n represents t or deletion (location: 21).
SEQ ID NO: 536: n represents an insertion of t (location: 21).
SEQ ID NO: 538: n represents t or deletion (location: 21).
SEQ ID NO: 539: n represents t or deletion (location: 21).
SEQ ID NO: 540: n represents t or deletion (location: 21).
SEQ ID NO: 541: n represents 21 to 37 times repetition of t (location: 21).
SEQ ID NO: 542: n represents 21 to 28 times repetition of a (location: 21).
SEQ ID NO: 543: n represents 8 to 10 times repetition of t (location: 21).
SEQ ID NO: 544: n represents 9 to 13 times repetition of a (location: 21).
SEQ ID NO: 545: n represents 9 to 11 times repetition of t (location: 21).
SEQ ID NO: 579: n represents t or deletion (location: 21).
SEQ ID NO: 580: n represents ca or deletion (location: 21).
SEQ ID NO: 581: n represents an insertion of a (location: 21).
SEQ ID NO: 583: n represents t or deletion (location: 21).
SEQ ID NO: 584: n represents ag or deletion (location: 21).
SEQ ID NO: 585: n represents 12 to 15 times repetition of t or deletion (location: 21).
SEQ ID NO: 586: n represents an insertion of t (location: 21).
SEQ ID NO: 588: n represents an insertion of aaa (location: 21).
SEQ ID NO: 590: n represents an insertion of c (location: 21).
SEQ ID NO: 592: n represents cct or deletion (location: 21).
SEQ ID NO: 593: n represents an insertion of g (location: 21).
SEQ ID NO: 595: n represents aatt or deletion (location: 21).
SEQ ID NO: 628: n represents an insertion of gat (location: 21).
SEQ ID NO: 630: n represents an insertion of t (location: 21).
SEQ ID NO: 635: n represents 12 to 15 times repetition of t (location: 21).
SEQ ID NO: 636: n represents 22 to 26 times repetition of a (location: 21).
SEQ ID NO: 657: n represents an insertion of t (location: 21).
SEQ ID NO: 659: n represents t or deletion (location: 21).
SEQ ID NO: 660: n represents an insertion of gtccactaaa (location: 21).
SEQ ID NO: 662: n represents an insertion of gtccactaaatgattgataattg (location: 21).
SEQ ID NO: 663: n represents an insertion of tgattgataattg (location: 21).
SEQ ID NO: 664: n represents a or deletion (location: 21).
SEQ ID NO: 665: n represents 16 to 18 times repetition of t (location: 21).
SEQ ID NO: 666: n represents g or deletion (location: 21).
SEQ ID NO: 670: n represents a or deletion (location: 21).
SEQ ID NO: 679: n represents 11 to 13 times repetition of a (location: 21).
SEQ ID NO: 680: n represents 10 to 12 times repetition of t (location: 21).
SEQ ID NO: 681: n represents 19 to 24 times repetition of a (location: 21).
SEQ ID NO: 682: n represents t or deletion (location: 21).
SEQ ID NO: 683: n represents 15 to 22 times repetition of t (location: 21).
SEQ ID NO: 684: n represents 7 to 9 times repetition of a (location: 21).
SEQ ID NO: 685: n represents 20 to 28 times repetition of a (location: 21).
SEQ ID NO: 693: n represents 11 to 14 times repetition of a (location: 21).
SEQ ID NO: 694: n represents 10 to 13 times repetition of c (location: 21).
SEQ ID NO: 696: n represents 18 to 21 times repetition of a (location: 21).
SEQ ID NO: 697: n represents 10 to 12 times repetition of a (location: 21).
SEQ ID NO: 698: n represents aaaat or deletion (location: 21).
SEQ ID NO: 699: n represents an insertion of gaaat (location: 21).
SEQ ID NO: 706: n represents 18 to 24 times repetition of a (location: 21).
SEQ ID NO: 712: n represents g or deletion (location: 21).
SEQ ID NO: 725: n represents 15 to 17 times repetition of a (location: 21).
SEQ ID NO: 726: n represents 15 to 21 times repetition of gt (location: 21).
SEQ ID NO: 727: n represents g or deletion (location: 21).
SEQ ID NO: 728: n represents t or deletion (location: 21).
SEQ ID NO: 729: n represents 9 to 11 times repetition of a (location: 21).
SEQ ID NO: 731: n represents 2 to 4 times repetition of t (location: 21).
SEQ ID NO: 733: n represents taag or deletion (location: 21).
SEQ ID NO: 739: n represents an insertion of cttt (location: 21).
SEQ ID NO: 742: n represents 8 to 9 times repetition of t (location: 21).
SEQ ID NO: 746: n represents tagacatttctta or gtagc (location: 21).
SEQ ID NO: 747: n represents 4 to 5 times repetition of a (location: 21).
SEQ ID NO: 753: n represents tg or deletion (location: 21).
SEQ ID NO: 762: n represents 8 to 9 times repetition of at (location: 21).
SEQ ID NO: 777: n represents 8 to 10 times repetition of t (location: 21).
SEQ ID NO: 779: n represents 6 to 7 times repetition of ca (location: 21).
SEQ ID NO: 781: n represents gttac or deletion (location: 21).
SEQ ID NO: 798: n represents t or deletion (location: 21).
SEQ ID NO: 808: n represents an insertion of at (location: 21).
SEQ ID NO: 810: n represents at or deletion (location: 21).
SEQ ID NO: 812: n represents 11 to 18 times repetition of t (location: 21).
SEQ ID NO: 817: n represents 8 to 9 times repetition of a (location: 21).
SEQ ID NO: 818: n represents gtgt or deletion (location: 21).
SEQ ID NO: 821: n represents 8 to 9 times repetition of a (location: 21).
SEQ ID NO: 823: n represents 10 to 12 times repetition of t (location: 21).
SEQ ID NO: 824: n represents 8 to 9 times repetition of t (location: 21).
SEQ ID NO: 833: n represents 6 to 7 times repetition of t (location: 21).
SEQ ID NO: 835: n represents 13 to 15 times repetition of gt (location: 21).
SEQ ID NO: 845: n represents 11 to 13 times repetition of t (location: 21).
SEQ ID NO: 846: n represents a or deletion (location: 21).
SEQ ID NO: 851: n represents 6 to 7 times repetition of a (location: 21).
SEQ ID NO: 859: n represents an insertion of cct (location: 21).
SEQ ID NO: 870: n represents 8 to 9 times repetition of t (location: 21).
SEQ ID NO: 876: n represents t or deletion (location: 21).
SEQ ID NO: 877: n represents an insertion of caggggctc (location: 21).
SEQ ID NO: 879: n represents 10 to 11 times repetition of a (location: 21).
SEQ ID NO: 886: n represents c or deletion (location: 21).
SEQ ID NO: 897: n represents ctccct or deletion (location: 21).
SEQ ID NO: 898: n represents an insertion of t (location: 21).
SEQ ID NO: 900: n represents ttttt or deletion (location: 21).
SEQ ID NO: 901: n represents an insertion of cc (location: 21).
SEQ ID NO: 903: n represents an insertion of agaaatttctagctgcctgcatttctagcagccca (location: 21).
SEQ ID NO: 913: n represents t or deletion (location: 21).
SEQ ID NO: 949: n represents t or deletion (location: 21).
SEQ ID NO: 950: n represents 8 to 9 times repetition of gtt (location: 21).
SEQ ID NO: 952: n represents c or deletion (location: 25).
SEQ ID NO: 964: n represents tt or deletion (location: 34).
SEQ ID NO: 971: n represents c or deletion (location: 21).
SEQ ID NO: 972: n represents an insertion of tt (location: 21).
SEQ ID NO: 974: n represents 3 to 30 times repetition of ga and 3 to 30 times repetition of gt (location: 21).
SEQ ID NO: 975: n represents g or deletion (location: 21).
SEQ ID NO: 982: n represents 7 to 8 times repetition of a (location: 21).
SEQ ID NO: 1005: n represents g or deletion (location: 21).
SEQ ID NO: 1006: n represents g or deletion (location: 21).
SEQ ID NO: 1007: n represents 12 to 14 times repetition of a (location: 21).
SEQ ID NO: 1008: n represents an insertion of t (location: 21).
SEQ ID NO: 1010: n represents 7 to 8 times repetition of ac (location: 21).
SEQ ID NO: 1028: n represents 12 to 15 times repetition of t (location: 21).
SEQ ID NO: 1031: n represents 11 to 13 times repetition of a (location: 21).
SEQ ID NO: 1032: n represents 10 to 12 times repetition of t (location: 21).
SEQ ID NO: 1033: n represents 19 to 24 times repetition of a (location: 21).
SEQ ID NO: 1035: n represents 11 to 14 times repetition of a (location: 21).
SEQ ID NO: 1036: n represents 18 to 21 times repetition of a (location: 21).
SEQ ID NO: 1043: n represents an insertion of a (location: 21).
SEQ ID NO: 1059: n represents an insertion of ca (location: 21).
SEQ ID NO: 1067: n represents aac or deletion (location: 21).
SEQ ID NO: 1068: n represents aac or deletion (location: 21).
SEQ ID NO: 1079: n represents an insertion of t (location: 21).
SEQ ID NO: 1081: n (location: 21) represents acctgtagcgctgcgctacccaa, and n (location: 22) represents an insertion of gatg or deletion.
SEQ ID NO: 1082: n represents an insertion of gatg or deletion (location: 21).
SEQ ID NO: 1083: n (location: 20) represents an insertion of acctgtagcgctgcgctacccaa, and n (location: 21) represents an insertion of gatg or deletion.
SEQ ID NO: 1084: n represents an insertion of acctgtagcgctgcgctacccaa (location: 20).
SEQ ID NO: 1089: n represents ttttcaattaggcaa or deletion (location: 21).
SEQ ID NO: 1090: n represents a or deletion (location: 21).
SEQ ID NO: 1091: n represents tttcttttcacaa or deletion (location: 21).
SEQ ID NO: 1093: n represents t or deletion (location: 21).
SEQ ID NO: 1094: n represents g or deletion (location: 21).
SEQ ID NO: 1101: n represents an insertion of t (location: 21).
SEQ ID NO: 1105: n represents an insertion of g or deletion (location: 23).
SEQ ID NO: 1107: n represents an insertion of t (location: 32).
SEQ ID NO: 1110: n (location: 19) represents an insertion of c or g, and n (location: 21) represents g or deletion.
SEQ ID NO: 1111: n (location: 10) represents an insertion of t or g, and n (location: 21) represents an insertion of t.
SEQ ID NO: 1112: n represents an insertion of t or g (location: 10).
SEQ ID NO: 1113: n represents a or deletion (location: 21).
SEQ ID NO: 1117: n represents c or deletion (location: 21).
SEQ ID NO: 1123: n represents gcccagctgg or deletion (location: 21).
SEQ ID NO: 1143: n represents an insertion of a (location: 21).
SEQ ID NO: 1161: n represents a or deletion (location: 21).
SEQ ID NO: 1162: n represents ttccttccac or deletion (location: 21).

## Claims

1. A method for detecting a genetic polymorphism(s), comprising creating oligonucleotide probes and/or oligonucleotide primers so that the probes and/or primers contain a polymorphic site(s) present in a gene encoding a receptor or a sequence complementary thereto or so that the polymorphic site(s) is/are contained in the amplified fragment when at least one of said gene encoding the receptor and said sequence complementary thereto is amplified; and detecting at least one genetic polymorphism in a gene of a subject encoding the receptor using the resultant oligonucleotide probes and/or oligonucleotide primers.

2. A method for detecting a genetic polymorphism(s) comprising creating oligonucleotide probes and/or oligonucleotide primers so that the probes and/or primers contain a polymorphic site(s) present in a gene encoding a receptor or a sequence complementary thereto or so that the polymorphic site(s) is/are contained in the amplified fragment when at least one of said gene encoding the receptor and said sequence complementary thereto is amplified; and detecting at least one genetic polymorphism in a gene of a subject encoding the receptor using the resultant oligonucleotide probes and/or oligonucleotide primers; wherein said polymorphic site is at least one of the polymorphic sites present in the nucleotide sequences as shown in SEQ ID NOS: 1 through 1168 or sequences complementary thereto.

3. A method for detecting a genetic polymorphism(s) comprising creating oligonucleotide probes and/or oligonucleotide primers so that the probes and/or primers contain a polymorphic site(s) present in a gene encoding a receptor or a sequence complementary thereto or so that the polymorphic site(s) is/are contained in the amplified fragment when at least one of said gene encoding the receptor and said sequence complementary thereto is amplified; and detecting at least one genetic polymorphism in a gene of a subject encoding the receptor using the resultant oligonucleotide probes and/or oligonucleotide primers; wherein said oligonucleotide probe and/or oligonucleotide primer is at least one selected from a group consisting of probes and primers having a polymorphic site-containing at least 13 nucleotide sequence within the nucleotide sequences as shown in SEQ ID NOS: 1 through 1168 or a sequence complementary to the polymorphic site-containing at least 13 nucleotide sequence.

4. The method according to claim 3 wherein the length of the oligonucleotide probe and/or oligonucleotide primer is from 13 to 60 nucleotides.

5. The method according to any one of claims 1 to 4 wherein information of the polymorphic site is as sown in Table 1.

6. The method according to any one of claims 1 to 5, wherein the oligonucleotide probe and/or oligonucleotide primer containing a polymorphic site is created so that the nucleotide positioned at its 5' or 3' end or its central part is the polymorphic site.

7. The method according to any one of claims 1 to 5, wherein the oligonucleotide probe containing a polymorphic site is composed of two fragments being linked to each other, one fragment being hybridizable to the gene encoding a receptor or the sequence complementary thereto and the other fragment being not hybridizable thereto, and said polymorphic site is positioned at the 5' or 3' end of the hybridizable fragment.

8. The method according to any one of claims 1 to 7, wherein the polymorphism is a single-nucleotide polymorphism, a polymorphism caused by deletion, substitution or insertion of a plurality of nucleotides, or a VNTR or microsatellite polymorphism.

9. A method for evaluating a drug, comprising evaluating from the detection results obtained by the method according to any one of claims 1 to 8 the efficacy and safety of the drug intermediated by the receptor.

10. A method for evaluating a drug, comprising evaluating from the detection results obtained by the method according to any one of claims 1 to 8 the degree of sensitivity of the drug intermediated by the receptor.

11. A method for selecting drugs, comprising selecting a drug to be used using the evaluation obtained by the method according to claim 9 or 10 as an indicator.

12. A method for selecting drugs, comprising comparing information about a polymorphism(s) in a gene encoding a receptor or a sequence complementary thereto with information about a polymorphism(s) in a gene encoding the receptor or a sequence complementary thereto obtained from a subject; analyzing individual differences regarding the efficacy and/or safety of drugs intermediated by the receptor; and selecting a drug to be used and/or a dose of the drug from the analysis results obtained.

13. The method according to any one of claims 1 to 12, wherein the receptor is at least one, selected from the group consisting of CD20, CD33, CSF3R, IL1R1, IL1R2, IL2R, HER2, IFNAR1, PGR, ACTH, ICAM1, VCAM1, ITGB2, PTGDR, PTGER1, PTGER2, PTGER3, PTGFR, GNA12, TBXA2R, BLTR2, CYSLT1, CYSLT2, PTAFR, BDKRB1, BDKRB2, ADRB1, ADRB2, HRH1, HRH2, HRH3, HTR3A, AGTR1, AGTRL1, AGTR2, AVPR1A, AVPR2, PTGIR, DRD1, ITGA2B, FOLR1, TNFR1, ADORA1, ADORA2A, ADORA2B, ADORA3, AVPR1B, ADRA1A, ADRA2A, ADRA2B, EDG1, EDG4, EDG5, GPR1, GPR2, GPR3, GPR4, GPR10, MC1R, MC2R, MC3R, MC4R, OXTR, SSTR1 and SSTR3.

14. An oligonucleotide created so that it contains a polymorphic site present in a gene encoding a receptor or a sequence complementary thereto.

15. An oligonucleotide created so that it contains a polymorphic site present in a gene encoding any receptor selected from the group consisting of CD20, CD33, CSF3R, IL1R1, IL1R2, IL2R, HER2, IFNAR1, PGR, ACTH, ICAM1, VCAM1, ITGB2, PTGDR, PTGER1, PTGER2, PTGER3, PTGFR, GNA12, TBXA2R, BLTR2, CYSLT1, CYSLT2, PTAFR, BDKRB1, BDKRB2, ADRB1, ADRB2, HRH1, HRH2, HRH3, HTR3A, AGTR1, AGTRL1, AGTR2, AVPR1A, AVPR2, PTGIR, DRD1, ITGA2B, FOLR1, TNFR1, ADORA1, ADORA2A, ADORA2B, ADORA3, AVPR1B, ADRA1A, ADRA2A, ADRA2B, EDG1, EDG4, EDG5, GPR1, GPR2, GPR3, GPR4, GPR10, MC1R, MC2R, MC3R, MC4R, OXTR, SSTR1 and SSTR3, or a sequence complementary thereto.

16. The oligonucleotide according to claim 14 or 15, which is created so that the nucleotide positioned at its 5' or 3' end or its central part is the polymorphic site.

17. The oligonucleotide according to claim 14 or 15, wherein the oligonucleotide containing a polymorphic site is composed of two fragments being linked to each other, one fragment being hybridizable to the gene encoding a receptor or the sequence complementary thereto and the other fragment being not hybridizable thereto, and said polymorphic site is positioned at the 5' or 3' end of the hybridizable fragment.

18. An oligonucleotide containing at least one polymorphic site present in the nucleotide sequences as shown in SEQ ID NOS: 1 through 1168 or sequences complementary thereto.

19. An oligonucleotide consisting of an at least 13 nucleotide sequence within any of the nucleotide sequences as shown in SEQ ID NOS: 1 through 1168, said at least 13 nucleotide sequence containing the 21^{st} nucleotide, or a sequence complementary to said at least 13 nucleotide sequence.

20. The oligonucleotide according to claim 19 having a length of 13-35 nucleotides.

21. An oligonucleotide selected from the group consisting of the nucleotide sequences as shown in SEQ ID NOS: 1 through 1168 and sequences complementary thereto.

22. An oligonucleotide which is designed in a genomic DNA region containing a polymorphic site in any of the nucleotide sequences as shown in SEQ ID NOS: 1 through 1168 or sequences complementary thereto so that it is located within 1000 bp of the polymorphic site toward the 5' and/or 3' end of the genomic DNA region, and which has a length of 13-60 nucleotides.

23. A microarray wherein the oligonucleotide according to any one of claims 14 to 22 is immobilized on a support.

24. A genetic polymorphism detection kit comprising the oligonucleotide according to any one of claims 14 to 22 and/or the microarray according to claim 23.
